# EUROPEAN PATENT SPECIFICATION

(11) **EP 0 428 620 B1**
(45) Date of publication and mention of the grant of the patent: **03.03.1999**
(21) Application number: 89910376.6
(22) Date of filing: 04.08.1989
(51) Int. Cl.: C08L 67/04, C08G 63/08

(54) **A method of plasticizing lactide polymers.**
Verfahren zur Weichmachung von Lactidpolymerisaten
Méthode pour la plastification de polymères de lactide.

(30) Priority: 08.08.1988 US 229896; 08.08.1988 US 229939; 08.08.1988 US 229894; 01.03.1989 US 317391; 31.07.1989 US 387678; 31.07.1989 US 387670; 31.07.1989 US 387676; 31.07.1989 US 386844
(43) Date of publication of application: 29.05.1991
(73) Proprietor: Biopak Technology, Ltd., Golden, Colorado 80403 (US)
(72) Inventor: SINCLAIR, Richard, G., Columbus, OH 43220 (US); PRESTON, Joseph, R., Radnor, OH 43066 (US)
(74) Representative: Mallalieu, Catherine Louise
(86) International application number: US8903380
(87) International publication number: WO9001521

(56) References cited:
- US-A- 2 703 316
- US-A- 4 677 191
- US-A- 4 728 721
- Chemical Abstracts, volume 110, no. 24, 12 June 1989, (Columbus, Ohio, US), Bodmeier R. et al : "The effect of the addition of low-molecular weightpoly(DL- lactide) on drug release from biodegradable poly(DL-lactide) drug delivery systems.", see page 409, abstract 219007f, & Int.J.Pharm. 1989, 51(1),1-8
- Chemical Abstracts, volume 108, no. 18, 2 May 1988, (Columbus, Ohio, US), Nakamura T. et al : "Surgical application of biodegradable films prepared from lactide--caprolactone copolymers.", see page 391, abstract 156432b, & Adv. Biomater 1987, 7(), 759-64
- Chemical Abstracts, volume 105, no. 19, 10 November 1986, (Columbus, Ohio, US), Chawla Attar S et al : "In-vivo degradation of poly(lactic acid) of different molecular weights.", see page 371, abstract 158776d, & Biomater.,Med.Devices, Artif.Organs 1986, 13(3), 153-62

## Description

### FIELD OF INVENTION

The present invention relates to processes for producing a biodegradable composition of polylactic acid and to a process of plasticizing a homopolymer.

### BACKGROUND OF THE INVENTION

There is a need for an environmentally biodegradable packaging thermoplastic as an answer to the tremendous amounts of discarded plastic packaging materials. U.S. plastic sales in 1987 were 53.7 billion pounds of which 12.7 billion pounds were listed as plastics in packaging. A significant amount of this plastic is discarded and becomes a plastic pollution that is a blot on the landscape and a threat to marine life. Mortality estimates range as high as 1-2 million seabirds and 100,000 marine mammals per year.

A further problem with the disposal of plastic packaging is the concern for dwindling landfill space. It has been estimated that most major cities will have used up available landfills for solid waste disposal by the early 1990's. Plastics comprise approximately 3 percent by weight and 6 percent of the volume of solid waste.

One other disadvantage of conventional plastics is that they are ultimately derived from petroleum, which leaves plastics dependent on the uncertainties of foreign crude oil imports. A better feedstock would be one that derives from renewable, domestic resources.

However, there are good reasons for the use of packaging plastics. They provide appealing aesthetic qualities in the form of attractive packages which can be quickly fabricated and filled with specified units of products. The packages maintain cleanliness, storage stability, and desirable qualities such as transparency for inspection of contents. These packages are known for their low cost of production and chemical stability. This stability, however leads to very long life of plastic, so that when its one time use is completed, discarded packages remain on, and in, the environment for incalculably long times.

The polymers and copolymers of lactic acid have been known for some time as unique materials since they are biodegradable, biocompatible and thermoplastic. These polymers are well behaved thermoplastics, 100 percent, truly biodegradable in an animal body via hydrolysis over a time period of several months to a year. In a wet environment they begin to show degradation after several weeks and disappear in about a year's time when left on or in the soil or seawater. The degradation products are lactic acid, carbon dioxide and water, all of which are harmless.

In practice, lactic acid is converted to its cyclic dimer, lactide, which becomes the monomer for polymerization. Lactic acid is potentially available from inexpensive feedstocks such as cornstarch or corn syrup, by fermentation, or from petrochemical feedstocks such as ethylene. Lactide monomer is conveniently converted to resin by a catalyzed, melt polymerization, a general process well-known to plastics producers. By performing the polymerization from an intermediate monomer, versatility in the resin composition is permitted. Molecular weight can be easily controlled. Compositions can be varied to introduce specific properties.

Homopolymers and copolymers of various cyclic esters such as glycolide, lactide, and the lactones have been disclosed in numerous patents and scientific publications. Early patents disclosed processes for polymerizing lactic acid, lactide, or both, and did not achieve high molecular weight polymers with good physical properties, and the polymer products were frequently tacky, sticky materials, without good physical properties. See, for example, U.S. Patents 1,995,970; 2,362,511; and 2,683,136. The Lowe patent, U.S. Patent 2,668,162 first teaches the use of pure glycolide and lactide to achieve high molecular weight polymers and copolymers of lactide. Lactide is the dilactone of lactic acid and is an internal ester of lactic acid. When lactide is formed, byproduct water is eliminated, permitting the lactide subsequently to be ring-opened polymerized to linear polyester of high molecular weight without tedious condensation methods. Copolymerization of lactide and glycolide imparted toughness and improved thermoplastic processability as compared to the homopolymers. Polymers and copolymers of excellent physical properties were obtained by using the intermediate, lactide, to form PLA. Copolymers of lactide and glycolide are disclosed by the Lowe patent which are tough, clear, cold-drawable, stretchable, and capable of forming at 210°C into self-supporting films.

Similar disclosures in the patent and other literature developed the processes of polymerization and copolymerization of lactide to produce very strong, crystalline, orientable, stiff polymers which were fabricated into fibers and prosthetic devices that were biodegradable and biocompatible, sometimes called absorbable. The polymers slowly disappeared by hydrolysis. See, for example, U.S. Patents 2,703,316; 2,758,987; 3,297,033; 3,463,158; 3,531,561; 3,620,218; 3,636,956; 3,736,646; 3,797,499; 3,839,297; 3,982,543; 4,243,775; 4,438,253; 4,496,446; European Patent Application 0146398, International Application WO 86/00533, and Offenlegundsschrift 2,118,127.

Other patents teach the use of these polymers as stiff surgical elements for biomedical fasteners, screws, nails, pins, and bone plates. See, for example, U.S. Patents 3,739,773; 4,060,089; and 4,279,249.

Controlled release devices, using mixtures of bioactive substances with the polymers and copolymers of lactide and/or glycolide, have been disclosed. See, for example, U.S. Patents 3,773,919; 3,887,699; 4,273,920; 4,419,340; 4,471,077; 4,578,384; 4,728,721; R.G. Sinclair, Environmental Science & Technology, 7 (10), 955 (1973). R.G. Sinclair, Proceedings, 5th International Symposium on Controlled Release of Bioactive Materials, 5.12 and 8.2, University of Akron Press, 1978. These applications of lactide polymers and copolymers required tough, or glassy materials, that were grindable and did not disclose physical properties for obvious use in thermoplastic packaging materials.

Some mention has been disclosed in the prior art for use of lactide copolymers for obvious packaging applications. Thus, in the aforementioned patent to Lowe, clear, self-supporting films are noted of a copolymer of lactide and glycolide. In U.S. Patent 2,703,316 lactide polymers are described as film formers, which are tough and orientable. "Wrapping tissue" was disclosed that was tough, flexible, and strong, brittle, or pliable. However, to obtain pliability the polylactide must be wet with volatile solvent, otherwise, stiff and brittle polymers were obtained. The lactide monomer is specified as having a melting point greater than 120°C. L-lactide monomer melts at 95°C and D,L-lactide melts at 128°C. This is an example of the prior art which teaches special modifications of lactide polymers to obtain pliability. Thus, in U.S. Patent 3,021,309, lactides are copolymerized with delta valerolactone and caprolactone to modify lactide polymers and obtain tough, white, crystalline solids. Soft, solid copolymer compositions are mentioned only with the copolymer of caprolactone and 2,4-dimethyl-4-methoxymethyl-5-hydroxypentanoic acid lactone, not with lactide compositions. U.S. Patent 3,284,417 relates to the production of polyesters which are useful as plasticizers and intermediates for the preparation of elastomers and foams. This patent excludes lactides and uses compositions based on 7 to 9 membered ring lactones, such as epsilon caprolactone, to obtain the desired intermediates. No tensile strength, modulus, or percent elongation data are given. U.S. Patent 3,297,033 teaches the use of glycolide and glycolide-lactide copolymers to prepare opaque materials, orientable into fibers suitable for sutures. It is stated that "plasticizers interfere with crystallinity, but are useful for sponge and films". Obvious in these disclosures is that the lactide polymers and copolymers are stiff unless plasticized. This is true also of U.S. Patent 3,736,646, where lactide-glycolide copolymers are softened by the use of solvents such as methylene chloride, xylene, or toluene. In U.S. Patent 3,797,499 copolymers of L-lactide and D,L-lactide are cited as possessing greater flexibility in drawn fibers for absorbable sutures. These fibers have strengths greater than 50,000 psi with elongation percentages of approximately 20 percent. Moduli are about one million psi. These are still quite stiff compositions compared to most flexible packaging compositions, reflecting their use for sutures. U.S. Patent 3,844,987 discloses the use of graft and blends of biodegradable polymers with naturally occurring biodegradable products, such as cellulosic materials, soya bean powder, rice hulls, and brewer's yeast, for articles of manufacture such as a container to hold a medium to germinate and grow seeds or seedlings. These articles of manufacture are not suitable for packaging applications.

U.S. Patent 4,620,999 discloses a biodegradable, disposable bag composition comprised of polymers of 3-hydroxybutyrate and 3-hydroxybutyrate/3-hydroxyvalerate copolymer. Lactic acid, by comparison, is 2-hydroxy propionic acid. U.S. Patent 3,982,543 teaches the use of volatile solvents as plasticizers with lactide copolymers to obtain pliability. U.S. Patents 4,045,418 and 4,057,537 rely on copolymerization of caprolactone with lactides, either L-lactide, or D,L-lactide, to obtain pliability. U.S. Patent 4,052,988 teaches the use of poly (p-dioxanone) to obtain improved knot tying and knot security for absorbable sutures. U.S. Patents 4,387,769 and 4,526,695 disclose the use of lactide and glycolide polymers and copolymers that are deformable, but only at elevated temperatures. European Patent Application 0108933 using a modification of glycolide copolymers with polyethylene glycol to obtain triblock copolymers which are taught as suture materials. As mentioned previously, there is a strong consensus that pliability is obtained in lactide polymers only by plasticizers which are fugitive, volatile solvents, or other comonomer materials.

Copolymers of L-lactide and D,L-lactide are known from the prior art, but citations note that pliability is not an intrinsic physical property. U.S. Patent 2,758,987 discloses homopolymers of either L- or D,L-lactide which are described as melt-pressable into clear, strong, orientable films. The properties of the poly-L-lactide are given as: tensile strength, 29,000 psi; percent elongation, 23 percent, tensile modulus 710,000 psi. The poly-D,L-lactide properties were: 26,000 psi tensile strength; 48 percent elongation; and a tensile modulus of 260,000 psi. Copolymers of L- and D,L-lactide, that is copolymers of L- and D,L-lactic acid, are disclosed only for a 50/50 by weight mixture. Only tack point properties are given (Example 3). It was claimed that one antipodal (optically active, e.g., L-lactide) monomer species is preferred for the development of high strength. The homopolymers of L-lactide and D,L-lactide, as well as the 75/25, 50/50, and 25/75, weight ratio, of L-/D,L-lactide copolymers are exampled in U.S. Patent 2,951,828 that discloses the bead polymerization of alpha-hydroxy-carboxylic acids. The copolymers have softening points of 110-135 C. No other physical property data are given relating to stiffness and flexibility except for physical properties relating to bead size and softening points in the 110°- 135°C range. The 95/5, 92.5/7.5, 90/10, and 85/15, weight ratio, of L-lactide/D,L-lactide copolymers are cited in U.S. Patents 3,636,956 and 3,797,499. They are evaluated as filaments from drawn fibers and have tensile strengths in excess of 50,000 psi, moduli of about one million, and percent elongations of approximately 20 percent. Plasticizers, the same as in U.S. Patent 3,636,956, above, were used to impart pliability. A snow-white, obviously crystalline polymer, is cited in Offenlegundsschrift 2,118,127 for a 90/10, L-lactide/D,L-lactide copolymer. No physical properties were given for this copolymer. The patent teaches the use of surgical elements.

U.S. Patents 3,297,033; 3,463,158; 3,531,561; 3,636,956; 3,736,646; 3,739,773; and 3,797,499 all disclose lactide polymers and copolymers that are strong crystalline, orientable polymers suitable for fibers and suture materials. These disclosures teach the use of highly-crystalline materials, which are oriented by drawing and annealing to obtain tensile strengths and moduli, typically, greater than 50,000 psi and 1,000,000 psi, respectively. Although formability is mentioned into a variety of shaped articles, physical properties of unoriented extrudates and moldings are not mentioned. For example, U.S. Patent 3,636,956 teaches the preparation of a 90/10 weight ratio of L-lactide/D,L-lactide and drawn, oriented fibers are cited. However, it is preferred in this disclosure to use pure L-lactide monomer for greater crystallinity and drawn fiber strength.

U.S. Patent 3,797,499 teaches the copolymerization of 95/5 weight ratio, of L-lactide/D,L-lactide (Example V); however, the material is formed into filaments. In column 5, line 1 Schneider teaches against enhanced properties in the range provided in the present invention. plasticizers such as glyceryl triacetate, ethyl benzoate and diethyl phthalate are used.

U.S. Patents 3,736,646; 3,773,919; 3,887,699; 4,273,920; 4,471,077; and 4,578,384 teach the use of lactide polymers and copolymers as sustained-drug release matrices that are biodegradable and biocompatible. Again, physical properties of the polymers from ordinary thermoforming methods such as film extrusion or molding are not mentioned.

Of particular interest, U.S. patent 4,719,246 teaches the blending of homopolymers of L-lactide, D-lactide, polymers or mixtures thereof; and copolymers of L-lactide or D-lactide with at least one nonlactide comonomer. The blending is intended to produce compositions having interacting segments of poly(L-lactide) and poly(D-lactide).

Canadian Patent 808,731 cites the copolymers of L- and D,L-lactide where a divalent metal of Group II is part of the structure. The 90/10, L-/D,L-lactide copolymer (Example 2) and the L-lactide homopolymer were described as "suitable for films and fibers". The 90/10 copolymer is described as a snow-white copolymer and the homopolymer of L-lactide can be molded to transparent films. (The more crystalline polymer should be the opaque, or snow-white material, which is the homopolymer.) The patent discloses "the fact that the novel polylactides of the present invention contain the metallic component of the catalyst in the form of a lactate is believed to be of significance". Furthermore, "the polylactides find utility in the manufacture of films and fibers which are prepared by conventional thermoplastic resin manufacturing methods". No physical property data are given on the strength and flexibility of the films.

Canadian Patent 863,673 discloses compositions of L-lactide and D,L-lactide copolymers in the ratios of 97/3, 95/5, 92.5/7.5, 90/10, and 85/15 ratios of L-/D,L-lactide, respectively. These were all characterized as drawn filaments for surgical applications. Tensile strength, approximately 100,000 psi, was high, elongation was approximately 20 percent and plasticizers were mentioned to achieve pliability. D,L-lactide compositions of less than 15 weight percent are claimed.

Canadian Patent 923,245 discloses the copolymers of L- and 0,L-lactide (Example 15). The 90/10 copolymer is described as a snow white polylactide. The polylactides prepared by the methods of the patent are stated to have utility in the manufacture of films or fibers prepared by conventional thermoplastic resin fabricating methods.

U.S. Patent 4,719,246 teaches the use of simple blending of poly L-and poly (D-lactide), referred to as poly (S-lactide) and poly (R-lactide), polymers of mixtures thereof; and copolymers of L-lactide or D-lactide with at least one nonlactide comonomer. The examples are all physical mixtures. The special properties of the "interlocking" stem from racemic compound formation (cf. "Stereochemistry of Carbon Compounds", E. L. Eliel, McGraw-Hill, 1962, p. 45). Racemic compounds consist of interlocked enantiomers, that is, the D and L forms (or R and S) are bonded to each other by polar forces. This can cause a lowering, or raising, of the crystalline melting points, depending on whether the D to D (or L to L) forces are less, or greater, than the 0 to L forces. Required of polymer racemic compounds to enhance the effect (and stated in U.S. Patent 4,719,246, Column 4, line 48) are homopolymers, or long chain lengths, of both D and L. The great symmetry or regularity of these structures permit them to fit together, or interlock, by very regular polar forces, either because they are the same, or mirror images. This leads to considerable crystallinity. The art of racemic compounds has a long history that goes back to classical chemistry.

U.S. patent 4,661,530, discloses the mixtures of a poly (L-lactic acid) and/or poly (D,L-lactic acid) and segmented polyester urethanes or polyether urethanes. Biodegradable materials are formed that are useful in synthetic replacements of biological tissues and organs in reconstructive surgery.

Nowhere in the prior art is it disclosed that lactide polymers, are capable of pliable, highly-extensible compositions by the use of lactide monomers, lactic acid, or oligomers of lactic acid or lactide as the plasticizer. None of the prior compositions are suitable for well-defined packaging needs of the thermoplastic polymers' industry.

It will be appreciated by those skilled in the art that duplicating the properties of one thermoplastic with another is not predictable. Thus, with crystal polystyrene, or OPS, there are exacting requirements for satisfactory performance of the polystyrene, which has been developed over many years to meet manufacturing and end-use specifications of OPS grades.

### BRIEF DESCRIPTION OF THE INVENTION

The present invention relates to a method for producing plasticized polymers of L-lactide, D-lactide, D,L-lactide and mixtures thereof suitable for packaging applications conventionally served by nondegradable plastics (e.g. polyethylene). The present invention further relates to a method for producing pliable films and other packaging items and to the unique product thereof. The invention has utility in producing a product that has the characteristics of the usual plastics yet is biodegradable.

Also, the invention relates to a process for preparing a material which is an offset, that is a replacement for crystal polystyrene, sometimes known as orientable polystyrene or OPS. The material is an offset for the OPS but is composed of a polyester capable of biodegrading in the environment over approximately 1 years time. The material is a polyester, comprised of polymerized lactic acid, prepared from either D-lactic acid or L-lactic acid, and D,L-lactic acid. The ratio of the two polymerized monomer units, the process treatment and in some cases certain adjuvants, determine the precise physical properties required for the exacting requirements of an OPS offset. Thus, at approximately a ratio of 90/10, L-lactic/D,L-lactic acid, the polymerized lactic acid (PLA) is a well behaved thermoplastic that is clear, colourless, and very stiff. As such it is very suitable for preparing films, foams, and other thermoformed items of disposable or one-way plastic. Having served its purpose as a packaging plastic, the PLA slowly environmentally biodegrades to innocuous products when left on or in the environment. This harmless disappearance can help alleviate the mounting problems of plastic pollution in the environment.

The general teaching of one aspect of the invention is that homopolymers of L-lactide, D-lactide, and D,L-lactide and copolymers of mixtures thereof that have been plasticized with lactide monomer(s), lactic acid or oligomers of lactide or of lactic acid have utility as well behaved thermoplastics which can mimic properties of the usual environmentally nondegradable plastics, (e.g., the properties of polyethylene and the like). These polymers preferably have the formula: 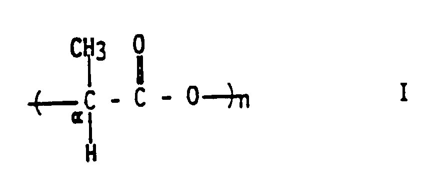 and are intimately plasticized with a plasticizer selected from lactide, lactic acid, oligomers of lactic acid, and mixtures thereof. Preferably n is 150 ≤ n ≥ 20,000. The oligomers of lactic acid further are preferably represented by the formula II, where m is an integer: 2 ≤ m ≤ 75. However, m is preferably 2 ≤ m ≤ 10. The plasticizer preferably comprises from 2 to 60 weight percent of the polymer. The polymers may be derived from monomers of lactide selected from L-lactide, D-lactide, meso D,L-lactide and mixtures thereof.

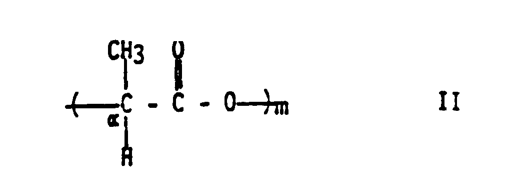

Lactide monomer can be present in an amount of from 5 to 40 weight percent of the polymer while lactide oligomer or lactic acid and its oligomers may be present in an amount of from 2 to 60 weight percent. This composition allows many of the desirable characteristics of polyethylene such as pliability, transparency, and toughness.

Further provided is a process for producing the biodegradable composition. The process includes the steps of mixing, heating, and melting one or more lactide monomers and catalyst; polymerizing the monomers of the solution to form a polymer at a temperature sufficiently low to allow the polymerization reaction to be stopped prior to complete polymerization; monitoring the level of monomer; and stopping the reaction prior to complete polymerization at an amount of monomer determined by the monitoring; so that unreacted monomer is trapped in association with the polymer.

Further provided is a process for producing a plasticised polymer of polyactic acid that comprises mixing, heating, and melting one or more lactide monomers and a catalyst; polymerizing the monomers of the solution to form a polymer without stopping the reaction; and incorporating plasticizer into the polymer whereby the plasticizer is selected from D-lactide, L-lactide, meso D,L-lactide, lactic acid, oligomers of lactic acid, and mixtures thereof.

The invention also provides a process for producing an environmentally biodegradable composition and an environmentally biodegradable composition useful as a replacement for polystyrene comprising polylactic acid units of the formula I where n is an integer between 75 and 10,000 and the alpha carbon is a mixture of L- and D-configurations with a preponderance of either D- or L- units, wherein the polymer is prepared from L-lactide or D-lactide, at 85 to 95 parts by weight, and D,L-lactide at 15 to 5 parts by weight, where the unoriented polymer has a tensile strength of at least 5000 psi and tangent modulus of at least 200,000 psi and dispersed plasticizer of 0.1 - 5 weight percent.

More particularly, according to one aspect of the present invention there is provided a process for producing an environmentally biodegradable composition of polylactic acid comprising:
a. preparing lactide monomer and catalyst;
b. polymerizing the monomer of step (a) to form a polymer at a temperature sufficiently low to allow the reaction to be stopped prior to complete polymerization;
c. monitoring the level of monomer in step (b); to determine the amount of monomer remaining;
d. stopping the polymerization of step (b) prior to complete reaction at an amount of monomer determined in step (c) so that unreacted monomer in a predetermined amount is trapped in association with the polymer; and the plasticizer is intimately dispersed in the polymer; wherein the predetermined amount of unreacted lactide is from 2 to 30 weight percent ; and
e. incorporating additional plasticizer into the composition whereby the additional plasticizer is further selected from L-lactide, D-lactide, meso D,L-lactide, lactic acid, oligomers of lactide, oligomers of lactic acid, and mixtures thereof; and the additional plasticizer is also intimately dispersed in the polymer.

According to another aspect of the present invention there is provided a process for producing a biodegradable composition of polylactic acid comprising:
a. preparing lactide monomer and catalyst;
b. polymerizing the monomer of the solution of step (a) to form a polymer; and
c. incorporating plasticizer into the polymer of step (b), whereby the plasticizer is selected from D-lactide, L-lactide, D,L-lactide, oligomers of lactic acid, oligomers of lactide, and mixtures thereof; and the plasticizer is intimately dispersed within the polymer.

According to a further aspect of the present invention there is provided a method of plasticizing a homopolymer of any one L-lactide, D-lactide, D,L-lactide or copolymers thereof or mixtures thereof, characterised in that any one of a lactide monomer, lactic acid, an oligomer of lactide, or an oligomer of lactic acid or mixtures thereof, is used as the plasticizer.

### BRIEF DESCRIPTION OF THE FIGURES

Figure 1 is a graph showing the relationship between percent lactide in the composition as plasticizer and tensile strength.

Figure 2 is a graph showing the relationship between percent lactide in the composition as plasticizer and elastic modulus.

Figure 3 is a graph showing the relationship between percent oligomer in the composition as plasticizer and tensile strength where curve A is for a 90/10 copolymer and curve B is for a 92.5/7.5 copolymer.

Figure 4 is a graph showing the relationship between percent oligomer in the composition as plasticizer and the elastic modulus where curve A is for a 90/10 copolymer and curve B is for a 92.5/7.5 copolymer.

Figure 5 illustrates the differential scanning calorimetry (DSC) plot of unannealed 90/10, L-/D,L-lactide copolymer of Example 5B.

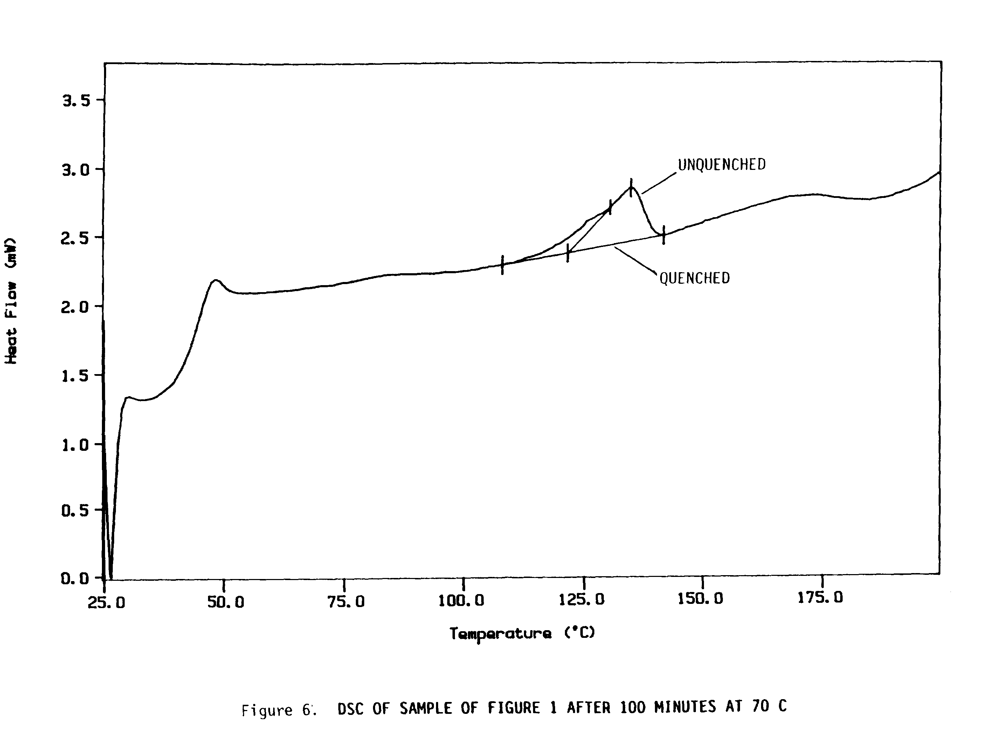

Figure 6 illustrates the DSC of the material of Example 5B after remaining at 70°C for 100 minutes.

Figure 7 illustrates the DSC of the material of Example 5B after annealing in 185°F overnight.

Figure 8 illustrates the DSC of the material of Example 5B that has been blended with 5 percent calcium lactate.

Figure 9 compares the melt viscosity versus shear rate characteristics of polystyrene and the lactide polymer prepared as in Example 8B.

Figure 10 illustrates a DSC for the copolymer of Example 8B.

Figure 11 illustrates a DSC for the L-lactide homopolymer that is added to the copolymer of Example 8B.

Figure 12 illustrates a OSC for the blended composition of Example 23 of the copolymer of Example 8B and a homopolymer of L-lactide.

### DETAILED DESCRIPTION OF THE INVENTION AND PREFERRED EMBODIMENTS

The environmentally biodegradable compositions disclosed herein are completely degradable to environmentally acceptable and compatible materials. The intermediate products of the degradation: lactic acid and short chain oligomers of lactide or lactic acid are widely distributed naturally occurring substances that are easily metabolized by a wide variety of organisms. Their natural end degradation products are carbon dioxide and water. Contemplated equivalents of these compositions such as those that contain minor amounts of other materials, fillers, or extenders can also be completely environmentally degradable by proper choice of materials. The compositions herein provide environmentally acceptable materials because their physical deterioration and degradation is much more rapid and complete than the conventional nondegradable plastics that they replace. Further, since all or a major portion of the composition will be polylactic acid, and/or a lactic acid derived lactide or oligomer, no residue or only a small portion of more slowly degrading residue will remain. This residue will have a higher surface area than the bulk product and an expected faster degradation rate.

The homopolymers of D-lactide, L-lactide, D,L-lactide as well as copolymers of D-lactide, L-lactide; D-lactide, D,L-lactide; L-lactide, D,L-lactide, and D-lactide, L-lactide, and D,L-lactide all produce materials useful in the invention when plasticized by lactide monomers, lactic acid, oligomers of lactide, oligomers of lactic acid, and mixtures thereof. A plasticizer may be produced by stopping the reaction before polymerization is completed, and additional plasticizer consisting of lactide monomers (D-lactide, L-lactide, D,L-lactide, or mixtures thereof), lactic acid, oligomers lactide or oligomers of lactic acid, and mixtures thereof is be added to the polymer. The polymer is defined by the formula: 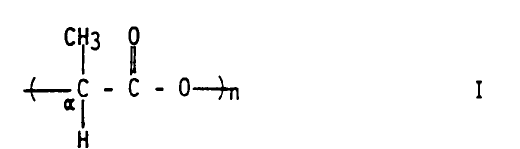 where n is the degree of polymerization (number of repeating units), and is plasticized with a plasticizer derived from incomplete polymerization of the monomers used to produce the polymer. The more intimately the plasticizer is integrated with the polymer the better are its characteristics. Additional monomer or oligomer is added to any residual monomer or oligomer remaining in the composition. The oligomers of lactic acid useful for a plasticizer are defined by the formula II, where m is an integer: 2 ≤ m < 75, however, the preferable range is: 2 ≤ m ≤ 10.

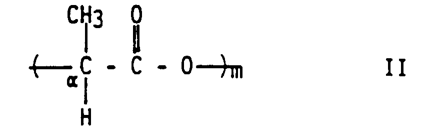

The proportions of L-lactide, D-lactide, and D,L-lactide are not critical to obtaining flexible thermoplastics. The parts of L-lactide, D-lactide, and D,L-lactide can vary over a wide, weight-ratio to form a homopolymer or copolymer. The lactide monomers employed in accordance with the invention are available commercially so that neither the monomeric reactant per se nor the method by which it is prepared constitute any portion of the invention.

D-lactide is a dilactone, or cyclic dimer, of D-lactic acid. Similarly, L-lactide is a cyclic dimer of L-lactic acid. Meso D,L-lactide is a cyclic dimer of D- and L-lactic acid. Racemic D,L-lactide comprises a mixture of D- and L-lactide. When used alone herein, the term "D,L-lactide" is intended to include meso D,L-lactide or racemic D,L-lactide.

One of the methods reported in the literature for preparing a lactide is to dehydrate lactic acid under high vacuum. The product is distilled at a high temperature and low pressure. Lactides and their preparation are discussed by W. H. Carothers, G. L. Dorough and M. J. Johnson (J. Am. Chem. Soc. 54, 761-762 [1932]); J. Gay-Lussac and J. Pelouse (Ann. 7, 43 [1833]); C. A. Bischoff and P. Walden (Chem. Ber. 26, 263 [1903]; Ann. 279, 171 [1984]); and Heinrich Byk (Ger. Pat. 267,826 [1912]); through Chem. Abstr. 8, 554, 2034 [1914]).

The optically active acids can be prepared by direct fermentation of almost any nontoxic carbohydrate product, by-product or waste, utilizing numerous strains of the bacterial genus Lactobacillus, e.g. Lactobacillus delbrueckii, L. salivarius, L. casei, etc. The optically active acids can also be obtained by the resolution of the racemic mixture through the zinc ammonium salt, or the salt with alkaloids, such as morphine. L-lactide is a white powder having a molecular weight of 144. If an impure, commercially-available product is employed in accordance with the present invention, it is preferable to purify it by recrystallization from anhydrous methyl isobutyl ketone. The snow-white crystals of L-lactide melt at 96-98°C.

D,L-lactic acid which is used in the preparation of D,L-lactide is available commercially. The D,L-lactic acid can be prepared synthetically by the hydrolysis of lactonitrile (acetaldehyde cyanohydrin) or by direct fermentation of almost any nontoxic carbohydrate product, by-product or waste, utilizing numerous strains of the bacterial genus Lactobacillus. D,L-lactide is a white powder having a molecular weight of 144. If an impure, commercially-available product is employed in accordance with the present invention, it is preferred to purify it by recrystallization from anhydrous methyl isobutyl ketone. One such commercially available product comprising a mushy semisolid melting at 90-130°C was recrystallized from methyl isobutyl ketone and decolorized using charcoal. After three such recrystallizations, the product was tumble-dried in vacuo under a nitrogen bleed for 8 to 24 hours at room temperature. The snow white crystals thus obtained comprise a D,L-lactide mixture melting from 115-128°C.

In preparing the compositions in accordance with the invention, it is preferred to carry out the reaction in the liquid phase in a closed, evacuated vessel in the presence of a tin ester of a carboxylic acid containing up to 18 carbon atoms. The compositions however, can also be prepared at atmospheric pressure with the polymerization system blanketed by an inert gas such as, for example, nitrogen. If polymerization is conducted in the presence of oxygen or air, some discoloration occurs with a resulting decrease in molecular weight and tensile strength. The process can be carried out at temperatures where the polymerization is sluggish in its later stages so as to trap residual monomer in the viscous polymer melt. Preferred temperatures for this purpose are generally between the melting points of pure L-lactide and pure D,L-lactide, or between 95 to 127°C. While in no way wishing to limit the scope of the invention it is presently believed that below about 129°C, the following occurs:
1. The lactide monomer mixture of L- and D,L-lactide monomers melt to form a eutectic mixture, which melts to a mobile fluid that is an intimate solution of one, two, or three monomers.
2. The fluid melt is polymerized by catalyst to form an increasingly viscous solution and eventually unreacted monomer is trapped in association with the polymer as a solution, rather than as a distinct heterogeneous phase. The monomer no longer can react since the reaction is extremely diffusion controlled and cannot contact the low concentration of active end-groups of the polymer.
3. The polymerization ceases or slows considerably so that at room temperature the blend of monomer and polymer are a solid solution that imparts plasticization, clarity, and flexibility to the composition.
4. The catalyst deactivates so that subsequent melt-fabrication does not reinitiate the polymerization.
5. The plasticized composition is quite stable since the residual monomer is very high boiling, e.g., lactide boiling point is 142°C at 8 torr, and is tightly associated with its open-chain tautomer, polylactide.

The process can be carried out at any temperature between the melting point of the L-lactide and 200°C and lactic acid or lactide is subsequently melt or solvent-blended into the polymer as a further processing step. Temperatures above 200°C are undesirable because of the tendency of the copolymer to be degraded. Increasing the temperature within the range of 95 to 200°C generally increases the speed of the polymerization. Good results are obtained by heating a mixture of L-lactide and D,L-lactide at a temperature between about 110°C and 160°C.

The catalysts employed in accordance with the invention are tin esters of carboxylic acids containing up to 18 carbon atoms. Examples of such acids are formic, acetic, propionic, butyric, valeric, caproic, caprylic, pelargonic, capric, lauric, myristic, palmitic, stearic and benzoic acids. Good results have been obtained with stannous acetate and stannous caprylate.

The catalyst is used in normal catalytic amounts. In general, a catalyst concentration in the range of about 0.001 to about 2 percent by weight, based on the total weight of the L-lactide and D,L-lactide is suitable. A catalyst concentration in the range of about 0.01 to about 1.0 percent by weight is preferred. Good results were obtained when the catalyst concentration is in the range of about 0.02 to about 0.5 percent by weight. The exact amount of catalyst in any particular case depends to a large extent upon the catalyst employed and the operating variables including time and temperature. The exact conditions can be easily determined by those skilled in the art.

The reaction time of the polymerization step, per se, is governed by the other reaction variables including the reaction temperature, the particular catalyst, the amount of catalyst and whether a liquid vehicle is employed. The reaction time can vary from a matter of minutes to a period of hours, or days, depending upon the particular set of conditions which are employed. Heating of the mixture of monomers is continued until the desired level of polymerization is detected. The level of polymerization can be determined by analysis for residual monomers. As discussed previously, the reaction temperature can be chosen to enhance the incorporation of monomer and provide plasticized compositions coming directly out of the polymerization reactor. The reaction can be halted at such time that the composition has attained the conversion of monomer to polymer that is desired to achieve the desired plastization. In the preferred embodiment of the invention, approximately 2 to 30 percent lactide is left unreacted, depending on the plasticization to be achieved.

In general it is preferred to conduct the polymerization in the absence of impurities which contain active hydrogen since the presence of such impurities tends to deactivate the catalyst and/or increase the induction time. It is also preferred to conduct the polymerization under substantially anhydrous conditions.

The copolymers prepared in accordance with the invention can be prepared by bulk polymerization, suspension polymerization or solution polymerization. The polymerization can be carried out in the presence of an inert normally-liquid organic vehicle such as, for example, aromatic hydrocarbons, e.g., benzene, toluene, xylene, ethylbenzene and the like oxygenated organic compounds such as anisole, the dimethyl and diethyl esters of ethylene glycol; normally-liquid saturated hydrocarbons including open chain, cyclic and alkyl-substituted cyclic saturated hydrocarbons such as hexane, heptane, cyclohexane, alkylcyclohexanes, decahydronaphthalene and the like.

The polymerization process can be conducted in a batch, semi-continuous, or continuous manner. In preparing the lactide monomeric reactants and catalyst for subsequent polymerization, they can be admixed in any order according to known polymerization techniques. Thus, the catalyst can be added to either of the monomeric reactants. Thereafter, the catalyst-containing monomer can be admixed with the other monomer. In the alternative, the monomeric reactants can be admixed with each other. The catalyst can then be added to the reactant mixture. If desired, the catalyst can be dissolved or suspended in an inert normally-liquid organic vehicle. If desired, the monomeric reactants either as a solution or a suspension in an inert organic vehicle can be added to the catalyst, catalyst solution or catalyst suspension. Still further, the catalyst and the monomeric reactants can be added to a reaction vessel simultaneously. The reaction vessel can be equipped with a conventional heat exchanger and/or a mixing device. The reaction vessel can be any equipment normally employed in the art of making polymers. One suitable vessel, for example, is a stainless steel vessel.

The environmentally biodegradable compositions produced in accordance with the present invention depending upon the L-lactide, D-lactide, meso D,L-lactide ratios, find utility in articles of manufacture, such as films, fibers, molding and laminates, which are prepared by conventional fabricating methods. These articles of manufacture are contemplated for nonmedical uses i.e. outside the body where they can substitute for the common environmentally nondegradable plastics.

Filaments, for example, are formed by melt-extruding the copolymer through a spinneret. Films are formed by casting solutions of the biodegradable compositions and then removing the solvent, by pressing solid biodegradable compositions in a hydraulic press having heated platens, or by extrusion through a die.

Various techniques including slow cooling and rapid cooling can be employed in preparing moldings from the copolymers of the invention.

Contemplated equivalents of the compositions prepared in accordance with the invention are those that contain minor amounts of other materials. The copolymers produced in accordance with the present invention can be modified, if desired, by the addition of a cross-linking agent, other plasticizers, a coloring agent, a filler and the like.

Cross-linking can be effected by compounding the compositions with free-radical initiators such as cumene hydroperoxide and then molding at elevated temperatures. This can improve heat-and solvent-resistance. Curing can also be effected by compounding the copolymers with multifunctional compounds such as polyhydric alcohols and molding, or thermoforming under heat and vacuum. Graft-extruder reactions to effect curing of the polyesters is an obvious method of cross-linking and chain-extending the copolymers.

In preparing moldings, a filler can be incorporated in the compositions prior to curing. A filler has the function of modifying the properties of a molding, including hardness, strength, temperature resistance, etc. Known filler materials include aluminum powder, powdered calcium carbonate, silica, kaolinite (clay), magnesium silicate and the like. Of particular advantage is starch, which blends well with the compositions to obtain a blend which is totally environmentally biodegradable. Other property modifications can be effected by melt blending the compositions with other polymers and copolymers of the lactides, glycolides, and caprolactone.

The compositions prepared according to the present invention can be used in producing reinforced laminates according to known procedures. In general, laminates are made from a fibrous mat or by assembling a multiplicity of sheets of material to form a matrix which is consolidated into a unitary structure by flowing molten precursor or composition through the fibrous material and curing it while in a mold or hydraulic press to form the polymer. Fibers which are used in forming the matrix include natural and synthetic fibers such as cellulose derived from wood, cotton, linen, hemp, and the like, glass, nylon, cellulose acetate and the like.

The compositions prepared in accordance with the invention and their preparation are further illustrated by the following specific examples.

### Example 1

### 80/20, L-lactide/racemic D,L-lactide

160 grams of L-lactide and 40 grams of racemic D,L-lactide, both of high purity (Purac, Inc., triply recrystallized), were charged into a 500 ml, round-bottom flask and purged with dry nitrogen overnight. 10 ml of stannous octoate is dissolved in 60 ml of anhydrous toluene, and 10 ml of the solvent is distilled to a Dean-Stark trap to effect dryness of this catalyst solution by azeotropic distillation. From the 10 ml of stannous octoate in 50 ml of dry toluene a 0.20 ml portion is removed with a syringe and injected into the lactides in the reaction flask. The nitrogen purge is continuous via a syringe needle connection that enters the reaction flask through a rubber septum and vents via a piece of tubing that connects to a bubbler. The nitrogen flow is maintained at 1-3 bubbles per second. The flask was heated in an oil bath maintained at 123-127°C. During the first part of the heating the lactides melt and are mixed thoroughly by swirling. Thereafter, the products become quite viscous. After 20 hours of heating, the flask and the colorless, transparent products are removed from the heating bath, cooled, the flask broken, and shocked with liquid nitrogen to remove glass from the product. The copolymer was molded in a heated hydraulic press. Compression molding to 5 to 10 mil thick films was possible at 20,000 lb pressure, at 170°C, in a time period of 2 minutes. The films were evaluated for their tensile properties on a Instron tester, and the results are listed in Table 1. Samples 1/8 inch thick were also molded for impact strength testing. A thermal gravimetric analysis (TGA) of the product was performed, noting the weight loss upon heating the sample to 150°C in 4 minutes and holding the temperature at 150°C for 60 minutes. The weight loss of the sample was 19.5 percent and nearly complete in 60 minutes. The weight loss is attributed to loss of lactide monomer. Results of differential scanning calorimetry (DSC) reveal that the composition has an endotherm beginning about 110°C, becoming more pronounced as the temperature increases to 200°C. No melting point was observed. Specimens were annealed at 185°F overnight and reexamined. They remained transparent, colorless and pliable. Samples of the copolymer could be remolded 6 times without any discoloration or obvious loss of strength. Thin films were clear, transparent, colorless, and quite flexible, despite the repeated molding.

**TABLE 1.**

| PROPERTIES OF COPOLYMERS^{(a)} OF L-LACTIDE AND D,L-LACTIDE WHEN PLASTICIZED BY LACTIDE | | | |
|---|---|---|---|
| Example No. | 1 | 2 | 3 |
| Film thickness, mil | 8 | 8 | 10 |
| | | | |
| Tensile strength, 1000 psi, ASTM 0638 | 3.9 | 1.7 | 7.9 |
| | | | |
| Elongation, percent | 28 | 806 | 3.5 |
| | | | |
| 100 percent modulus, 1000 psi | 0.74 | -- | -- |
| | | | |
| 200 percent modulus, 1000 psi | 1.20 | -- | -- |
| | | | |
| Tangent modulus, 1000 psi | 36.6 | -- | 289 |
| | | | |
| Izod impact strength, ft-lb/in.^{(b)} | 0.63 | -- | 0.4 |
| | | | |
| M_{w}, 1000's | 540 | 281 | 341 |
| | | | |
| Mₙ, 1000's | 270 | 118 | 97.5 |
| | | | |
| Residual lactide,^{(c)} percent | 19.5 | 27.8 | 2.7 |

| | | | |
|---|---|---|---|
| (a) 80/20, weight ratio, of L-/racemic D,L-lactide. | | | |
| (b) 1/8 inch, notched samples. | | | |
| (c) By isothermal TGA weight loss at 150°C. | | | |

### Example 2

In a 3-liter, round-bottom flask was charged 1.84 Kg of L-lactide, 0.46 Kg of racemic D,L-lactide and 2.3 ml of the stannous octoate solution, similar to Example 1. The mixture was purged with argon for 3 hours, then heated isothermally in a 125°C oil bath. The mixture melts, was mixed thoroughly by swirling, and forms a homogeneous, transparent, colorless fluid whose viscosity increases substantially after several hours. After 64 hours the flask was removed from the heating bath, cooled, and the glass removed from the clear, transparent, solid product. The rubbery composition was guillotined into slices and ground to 1/8 inch, or smaller, size in a grinder with dry ice. The grind was dried in an air circulating oven at 100°F for several hours, then vacuum dried overnight at ambient temperature. Compression-molded films were prepared as described in Example 1 and the films were examined for their tensile properties and weight loss by TGA as shown in Table 1.

### Example 3

In a 250-ml, round bottom flask was placed 79.98 g of L-lactide, 20.04 g of racemic D,L-lactide, and 0.20 ml of stannous octoate solution, similar to Example 1. The flask was swept by nitrogen through inlets and outlets and heated in a 125°C oil bath. The mixture melted to a colorless and fluid liquid that was thoroughly mixed by swirling the flask. After 2 hours, the oil bath temperature was increased to 147° C, and after 14 hours total heating time, the temperature was decreased to 131°C. Total heating time was 18 hours. The product is transparent, colorless, and glassy. It was evaluated, similar to the preceding examples and the results are recorded in Table 1.

Examples 1 to 3 reveal the effect of reaction temperature on the properties of the copolymers as occasioned by the resulting composition.

### Example 4

Films of the copolymers of Examples 1 and 3 were immersed in water for several months. After 3 weeks, the copolymer of Example 1 became hazy while that of Example 3 remained clear for approximately 2 months; after 3 months the film of Example 3 became noticeably hazy and the film of Example 1 is white and opaque. The water that had been in contact with the film of Example 1 tastes acidic while that of Example 3 is tasteless.

Inspection of the data of Table 1 reveals that the copolymer of Example 1 is an environmentally biodegradable replacement for polyethylene. Those skilled in the art will recognize that the physical properties of the copolymer are an excellent combination useful for many packaging applications. Its tensile strength and initial tangent modulus compare favorably with polyethylene compositions used, for example, in plastic trash bags, general film wrap, plastic shopping bags, sandwich wrap, six pack yokes and the like. The shape of the stress-strain curves are approximately the same for both the copolymer and that for a linear low density polyethylene composition commonly used in trash bag compositions. A comparison of properties are shown in Table 2.

**TABLE 2.**

| COMPARISON OF POLYETHYLENE TO POLYLACTIC ACID POLYMERS | | | |
|---|---|---|---|
| Property | LDPE-^{(a)} NA 272 | LLDPE^{(b)} | Lactide Copolymer^{(c)} |
| Tensile strength, 1000 psi, ASTM Standard C | 2.18 | 2.9 | 3.90 |
| | | | |
| Elongation, % | 261 | 500 | 280 |
| | | | |
| Tangent modulus, 1000 psi | 54.9 | 51.0 | 36.6 |
| | | | |
| 100% modulus, 1000 psi | 1.77 | -- | 0.74 |
| | | | |
| 200% modulus | 1.82 | -- | 1.20 |
| | | | |
| HDT,^{(d)} 264 psi, °F | 95 | 99 | 122 |

| | | | |
|---|---|---|---|
| (a) Linear low density polyethylene, 5-10 mil, 2-in./min., our experiments. | | | |
| (b) Linear low density polyethylene, data from computer file. | | | |
| (c) Copolymer of L-lactide/racemic D,L-lactide, Example 1. | | | |
| (d) Heat deflection temperature. | | | |

The lactide polymerization can be stopped at incomplete monomer-to-polymer conversion in a controllable fashion. This is illustrated in Examples 1 and 2. The lactide monomer binds very intimately with polymers of lactides. Alternatively, the compositions can be derived by mixing of lactide with preformed polymer. In that case, the lactide added can be the same or different with respect to stereochemistry, i,e., L-, D-, or D,L-lactide to that used to make the polymer.

The compounding can be accomplished by blending the molten polymer with lactide monomer in conventional processing equipment such as a mill roll or a twin screw compounder. The normally stiff, glassy, lactide polymers are flexibilized by the lactide and remain transparent, colorless, and very nearly odorless. The lactide is not very fugitive, requiring heating, and a nitrogen sweep, typically, 170-200°C for 20-60 minutes to remove the lactide in a gravimetric analysis. Neither is the lactide visible in films under an optical microscope. The lactide domains are submicron in size. This flexibilizing of the polylactic acid suggests its use as a environmentally biodegradable replacement for polyolefin, disposable, packaging films.

### Examples 5-16

A series of experiments were performed in which copolymers of L-and racemic D,L-lactide were prepared, melt blended with variable amounts of lactide, and the physical properties of the blends evaluated as a function of the lactide composition. Monomer lactide content was assayed by a previously developed isothermal, thermogravimetric analysis (TGA). The lactide contents were measured before and after compounding and molding into films.

It was observed that open roll, 2 roll, milling tended to volatilize the lactide at temperatures required for the very high, molecular weight lactide copolymers. These losses could be minimized by masterbatching or by using lower molecular weight lactide copolymers (and their lower attendant mixing temperatures). A better mixing and blending method was a conventional, twin screw extruder, which minimized volatile losses. Some results are shown in Table 3. The blends of polylactide and lactide plasticizer are quite pliable, becoming increasingly so with increasing lactide content. They are colorless and transparent. Only a very faint (pleasant) odor of lactide is detectable and no discernable taste of lactide was noticeable. The Table 3 plasticized film samples were tear resistant, easily foldable, and can be punctured without shattering or tearing. They stiffen somewhat when placed in a cooler (5 C, 40°F), but remain flexible and creasible without breaking. These films noticeably soften in the hand, indicating a glass transition temperature below 37°C. When the lactide content is less than 20 percent, the films will have a rattle typical of a polyolefin film. At greater lactide contents the films have the drape and "warm" feel of a PVC.

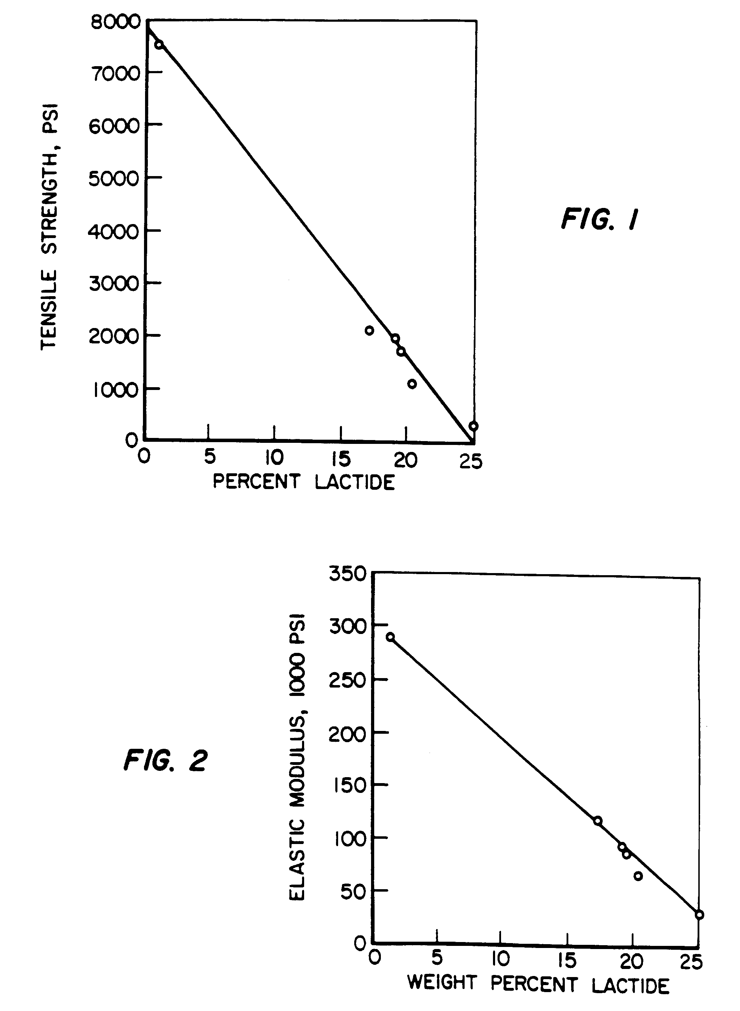

As shown in Table 3, the elastic moduli (initial tangent moduli) can be relatively high, similar to a linear low density polyethylene (LLDPE). This is an indication of potential form stability. Lower moduli and tensile strengths are similar to low density polyethylene (LDPE). Physical properties, as a function of lactide content, were plotted as shown in Figures 1 and 2. Referring to Table 3, at approximately 17-20 percent lactide content, the tensile properties are similar to polyethylenes used in trash bags and shopping bags.

At lower lactide contents, the blends have a similarity to polypropylene. Some data can be compared in Table 3. Table 4 defines the conventional plastics used in the comparisons.

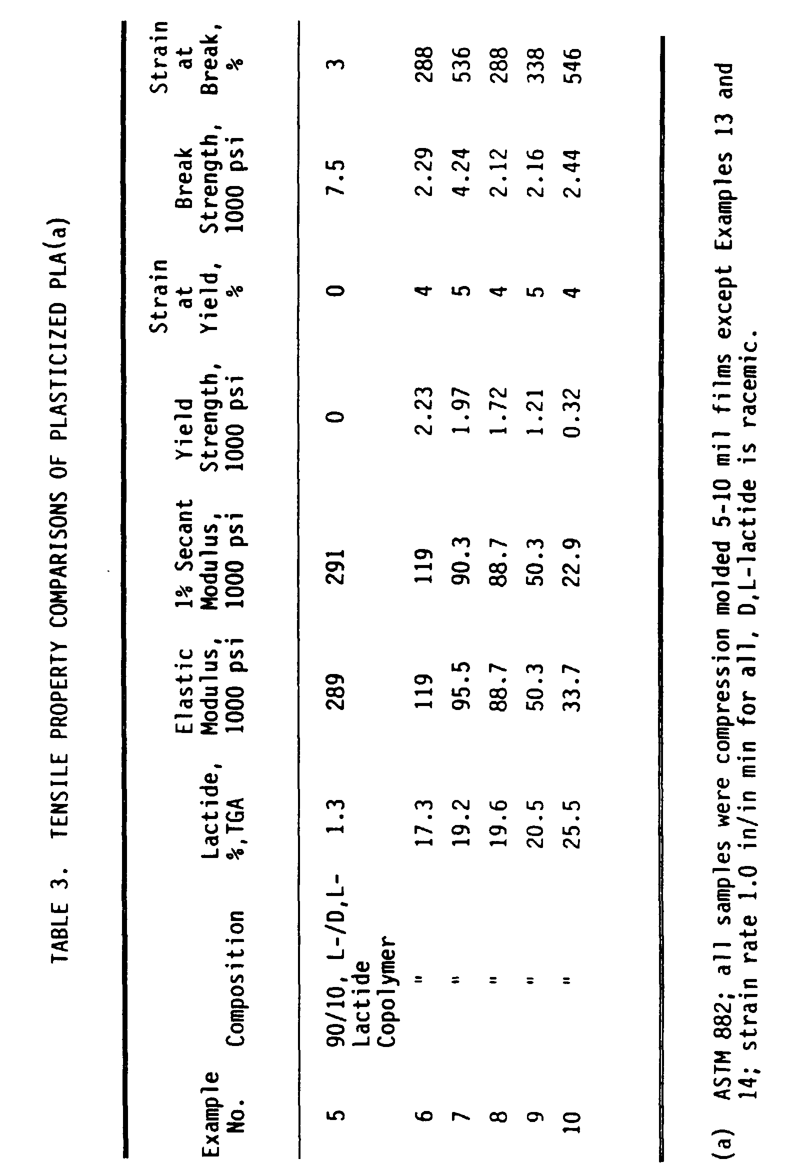

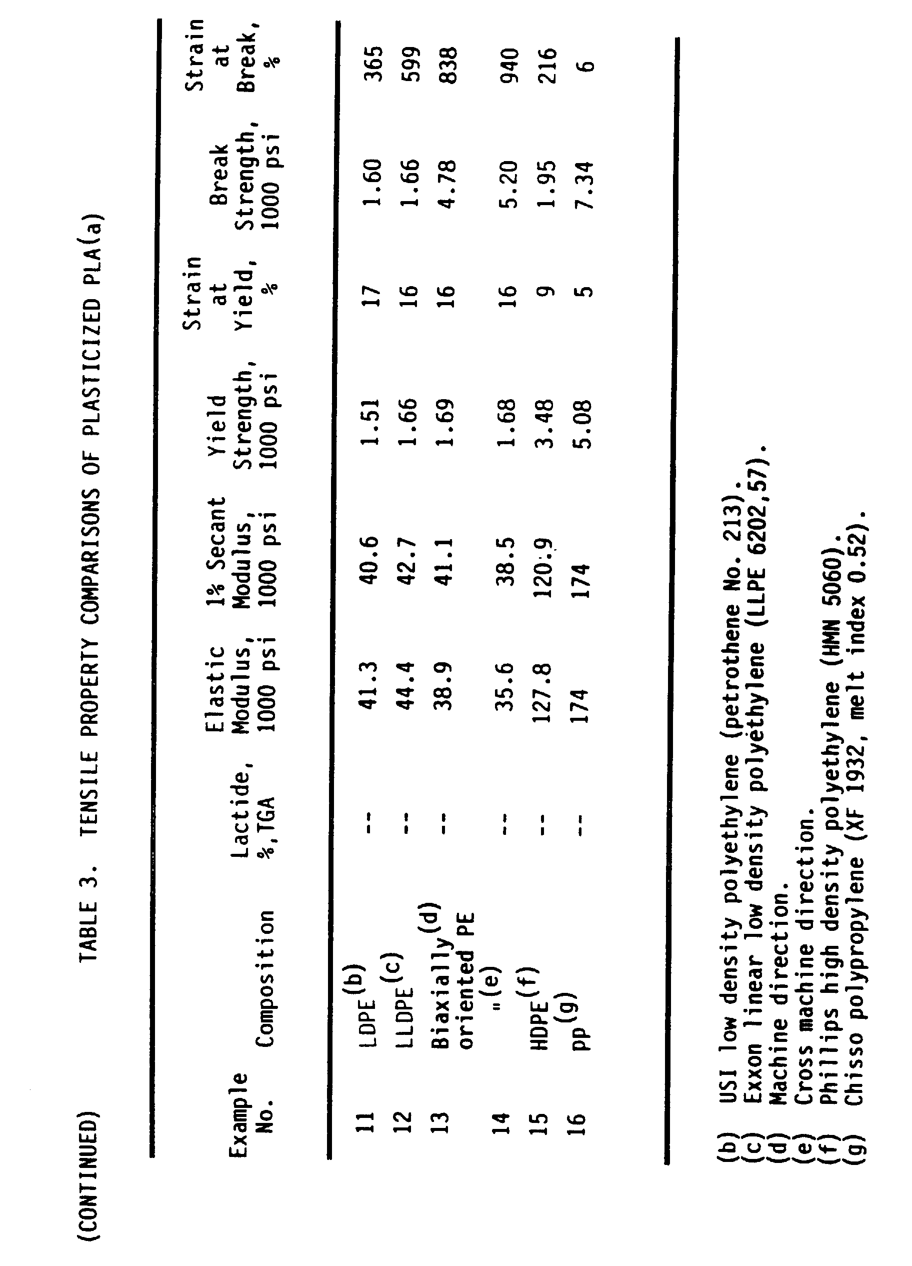

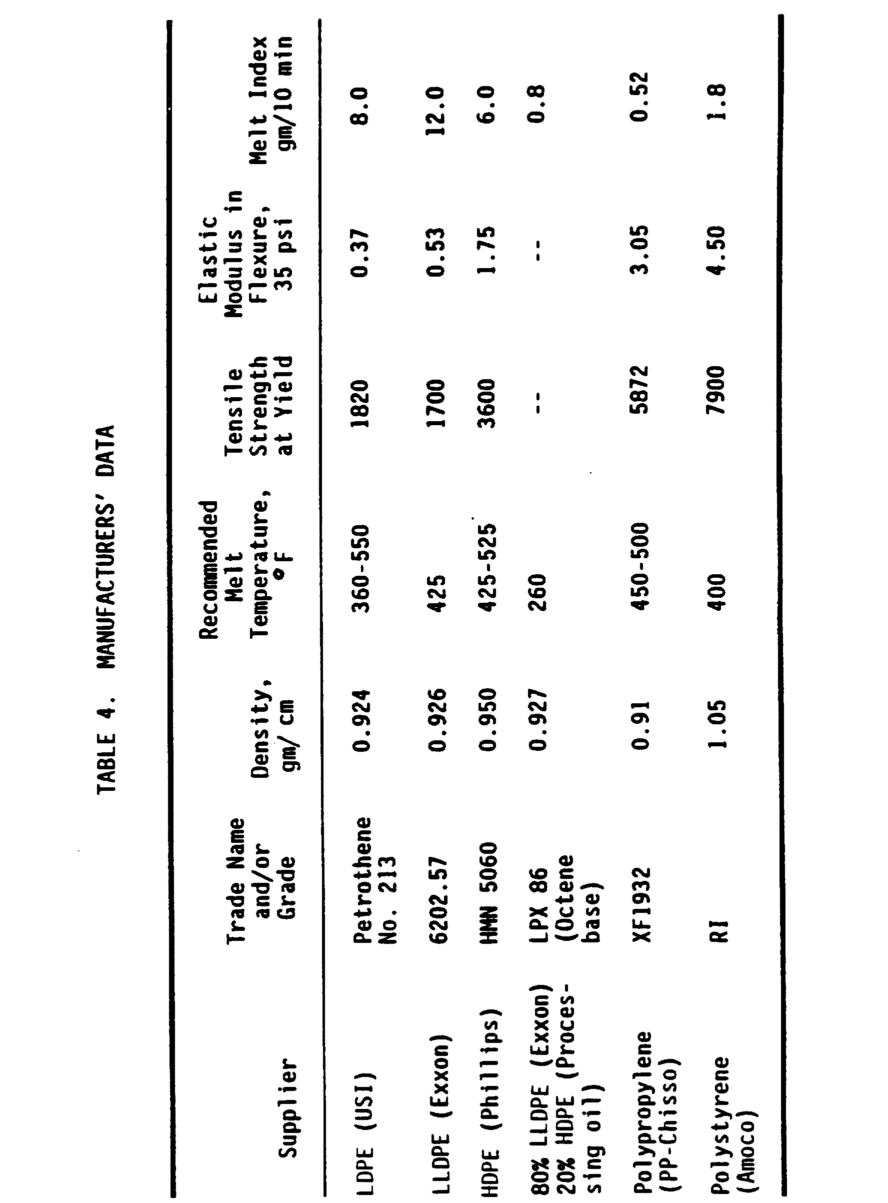

Table 3 reveals some data for lactide and polylactide mixtures. The results do not differ remarkably from similar compositions of Examples 1 and 2, prepared by other means. However, those skilled in the art will recognize that the precise physical properties will vary somewhat depending on the intimacy of the mixture, the tensile testing conditions, and the fabrication technique for preparing the films. Comparisons from Table 3 reveal that the lactide-polymer mixtures have a broad range of controllable compositions that mimic many conventional, nondegradable plastic types.

### Example 17

An oligomeric polylactic acid (OPLA) was prepared for mixing with polylactides as follows. An 88 percent solution of L-lactic acid (956 g) was charged to a 3-neck flask (1 liter) fitted with a mechanical stirrer and a pot thermometer. The reaction mixture was concentrated under a nitrogen purge at 150-190°C at 200 mm Hg for 1 hour until the theoretical water of dilution was removed. No catalyst was used except for lactic acid and its oligomers. This temperature and vacuum were maintained and distillation continued for 2 hours until 73 percent of the theoretical water of dehydration was removed.

The total time required was 3 hours. At this time the reaction was stopped. The water samples and the pot oligomer were titrated with 0.5N NaOH. Some lactic acid, 26.2 g, was found in the water distillate. The pot oligomer (OPLA) was also refluxed with excess 0.5N NaOH, then back titrated with standard H₂SO₄. The data are recorded in Table 5. The OPLA flows well when hot, and shows some cold flow. It has a degree of polymerization of 3.4. It was used in Example 20 where it was melt blended with the polymer of Example 19.

**TABLE 5.**

| CHARACTERIZATION OF OPLA OF EXAMPLE 1 | | | | |
|---|---|---|---|---|
| Percent Dehydrated, Theoretical | Titratable Acid, percent | Titratable Ester, percent | Total Expressed as Lactic Acid percent | Degree of Polymerization |
| 58 | 34.4 | 82.4 | 116.8 | 3.4 |

### Example 18

The procedure of Example 17 was repeated except the distillation was conducted more slowly. After 8 hours of heating during which the temperature was slowly advanced from 63 to 175°C at 200 mm Hg, a sample of the pot was titrated to reveal 62.2 percent of theoretical water removal. Titration revealed a degree of polymerization of 4.3. The molecular weight of the OPLA was further advanced over 2 hours by heating at 179°C and using a vacuum pump. The OPLA was no longer soluble in 0.1N NaOH, was water white, and would cold flow. This material is a second example of an OPLA preparation with somewhat higher degree of polymerization as compared to Example 1. It was mixed with polylactide in Examples 22 and 25. It is estimated that the degree of polymerization was about 6-10.

### Example 19

A polymer of lactide was prepared by methods similar to Example 3. A 90/10, weight percent L-/racemic D,L-lactide copolymer was melt polymerized using 0.02 parts per hundred, anhydrous stannous octoate catalyst. In a similar manner a 100 percent L-lactide homopolymer (L-PLA) was prepared. The copolymer was melt blended with the homopolymer at 350°F in a twin-screw extruder at a weight ratio of 90/10, copolymer/homopolymer. Gel permeation chromatography (GPC) of the blend reveals a weight-average molecular weight (M_{w}) of 182,000 and a number-average molecular weight (Mₙ) of 83,000. Residual lactide monomer by thermogravimetric analysis (TGA) was 1.7 weight percent. This blend was mixed with the oligomeric polylactic acid of (OPLA) of Example 17 to provide Example 20. The tensile properties are listed in Table 6.

### Example 20

The polymer of Example 19 was melt blended with the OPLA of Example 17 on an open, 2-roll, mill for 20 minutes at 325°F. The mix was compression molded into films and tested as shown in Table 6. The GPC molecular weights were smooth, monomodal distributions (M_{w}/Mₙ = 2.6) with M_{w} = 192,000 and Mₙ = 73,000.

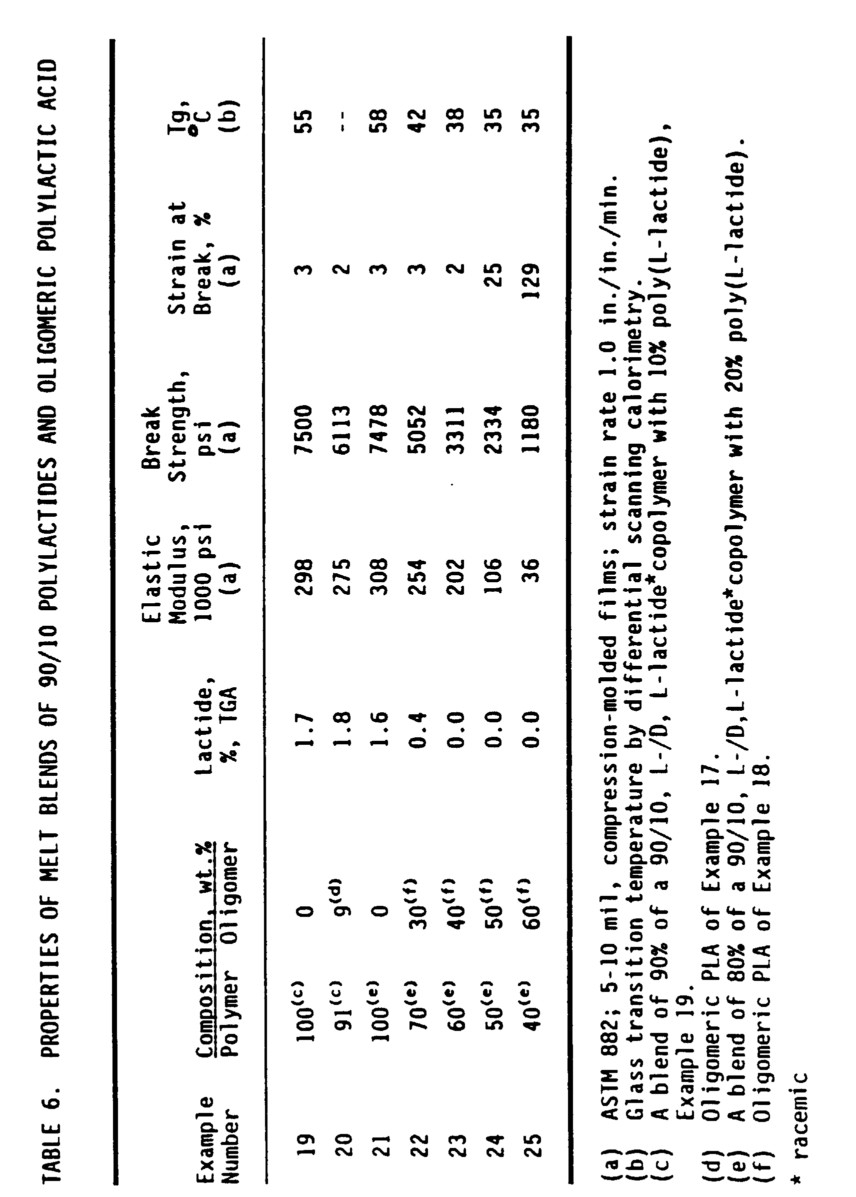

### Example 21-25

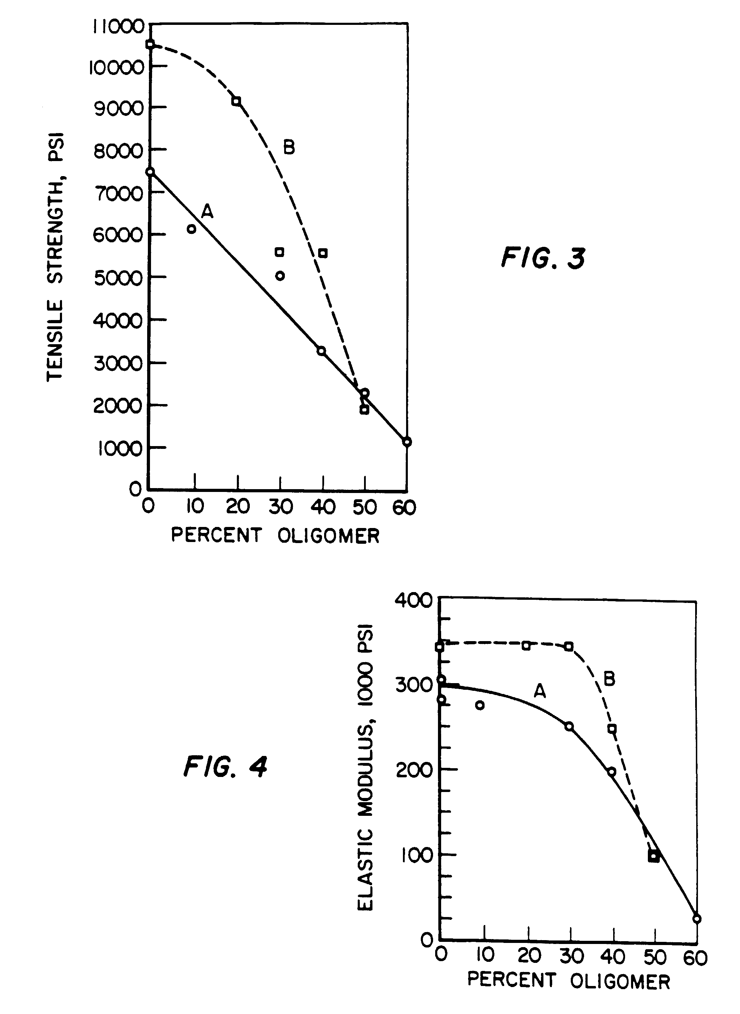

The copolymer of Example 19 was melt blended with 20 percent of the L-PLA described in Example 19. The blend is listed as Example 21 in Table 6, where its analyses and tensile properties are listed. Example 21 was, in turn, melt blended with various amounts of the OPLA of Example 18 and these were tested as before and listed in Table 6, Examples 22 to 25. Table 7 lists the GPC molecular weights of these compositions. The tensile strengths and moduli are compared to the weight percentages of OPLA in Figures 3 and 4 (Lower Curves).

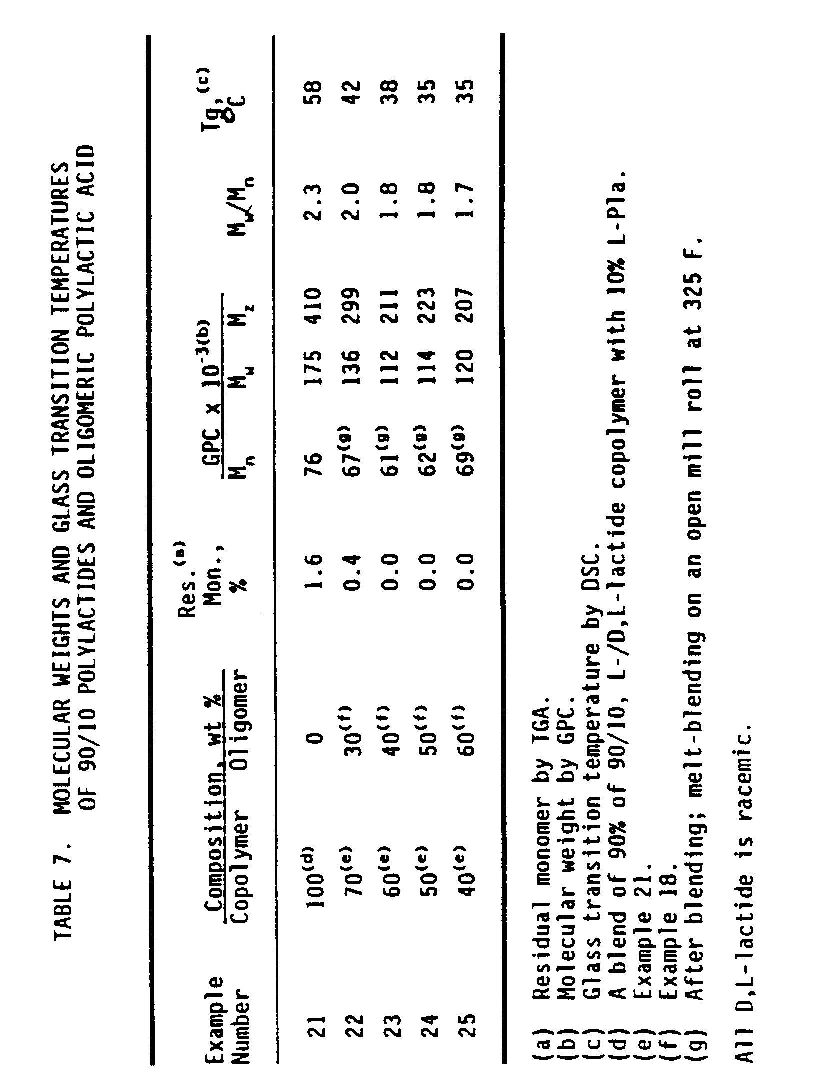

### Examoles 26-30

A second series of copolymers was blended with the OPLA. A 92.5/7.5, L-/D, L-lactide copolymer was prepared by methods similar to Examples 19 and 21. This is Example 26 of Tables 8 and 9. It was melt blended with the OPLA of Example 18 on an open, 2-roll mill at 325°F for approximately 20 minutes. The blends were compression molded into 3-5 mil thick films and their tensile properties and GPC molecular weights measured. The properties are recorded in Tables 8 and 9, and plotted in Figures 3 and 4. The second series of blends revealed significantly higher values for the tensile properties although the molecular weights were lower. This may be due to lower residual lactide monomer and/or the change in high polymer composition. All of the OPLA polylactide blends could be easily molded into tack free, transparent films.

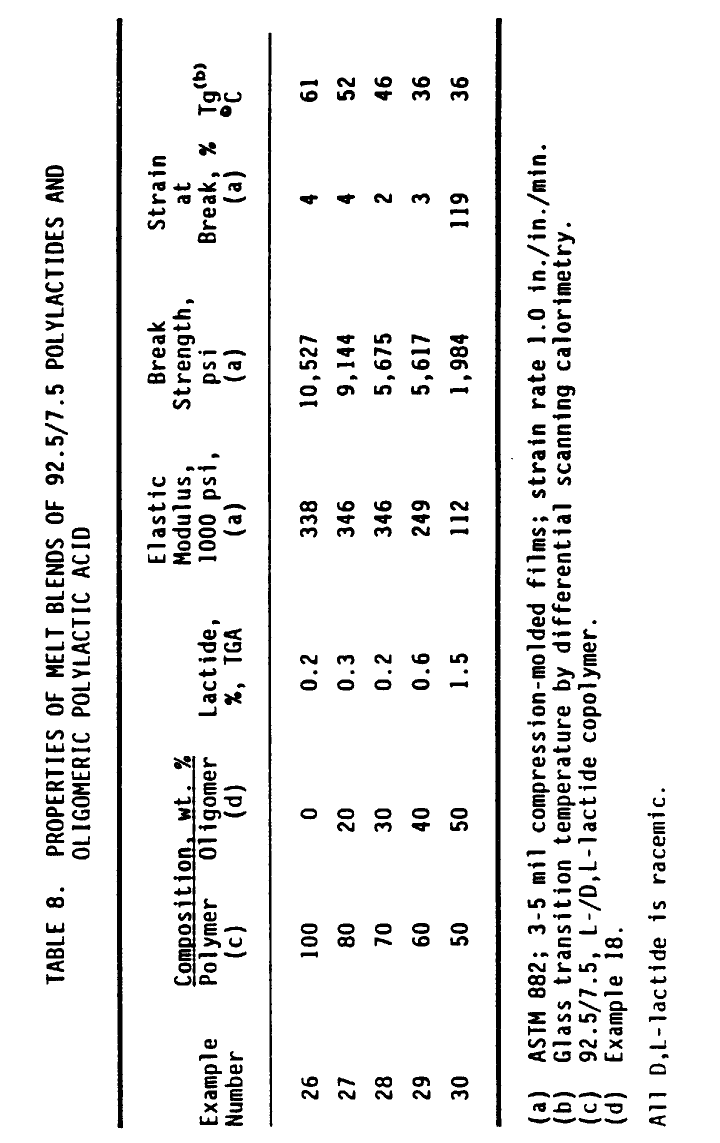

**TABLE 9.**

| MOLECULAR WEIGHTS OF 9.25/7.5, L-/RACEMIC D,L-LACTIDE COPOLYMERS | | | | | |
|---|---|---|---|---|---|
| Example No. | % OPLA | GPC x 10^{-3(a)} | | | |
| | | Mₙ | M_{w} | M_{z} | M_{w}/Mₙ |
| 26 | 0 | 63 | 124 | 228 | 1.95 |
| 27 | 20 | 60 | 108 | 189 | 1.81 |
| 28 | 30 | 48 | 80 | 125 | 1.66 |
| 29 | 40 | 59 | 96 | 151 | 1.65 |
| 30 | 50 | 56 | 92 | 141 | 1.64 |

| | | | | | |
|---|---|---|---|---|---|
| (a) GPC | | | | | |

### Examples 31 and 32

Film specimens with, and without plasticizer were exposed to seawater at Daytona, Florida from March through May. The pH of the water varied from 7.3 to 7.6 and the salinity from 33.2 to 38.4 ppt. The water gradually warmed in the test from 15 to 27°C. The specimens were cut into strips and tensile tested before, and after, periodic intervals in the seawater. The results are shown in Table 10. All of the samples showed whitening and physical degradation, which became progressive with time. Without plasticizer the samples showed whitening and degradation after six weeks in the seawater. The OPLA polylactide blend degraded faster, revealing clear evidence of degradation after 3 weeks. The incorporation of 20 percent lactide provoked immediate whitening and obvious degradation after one week of exposure.

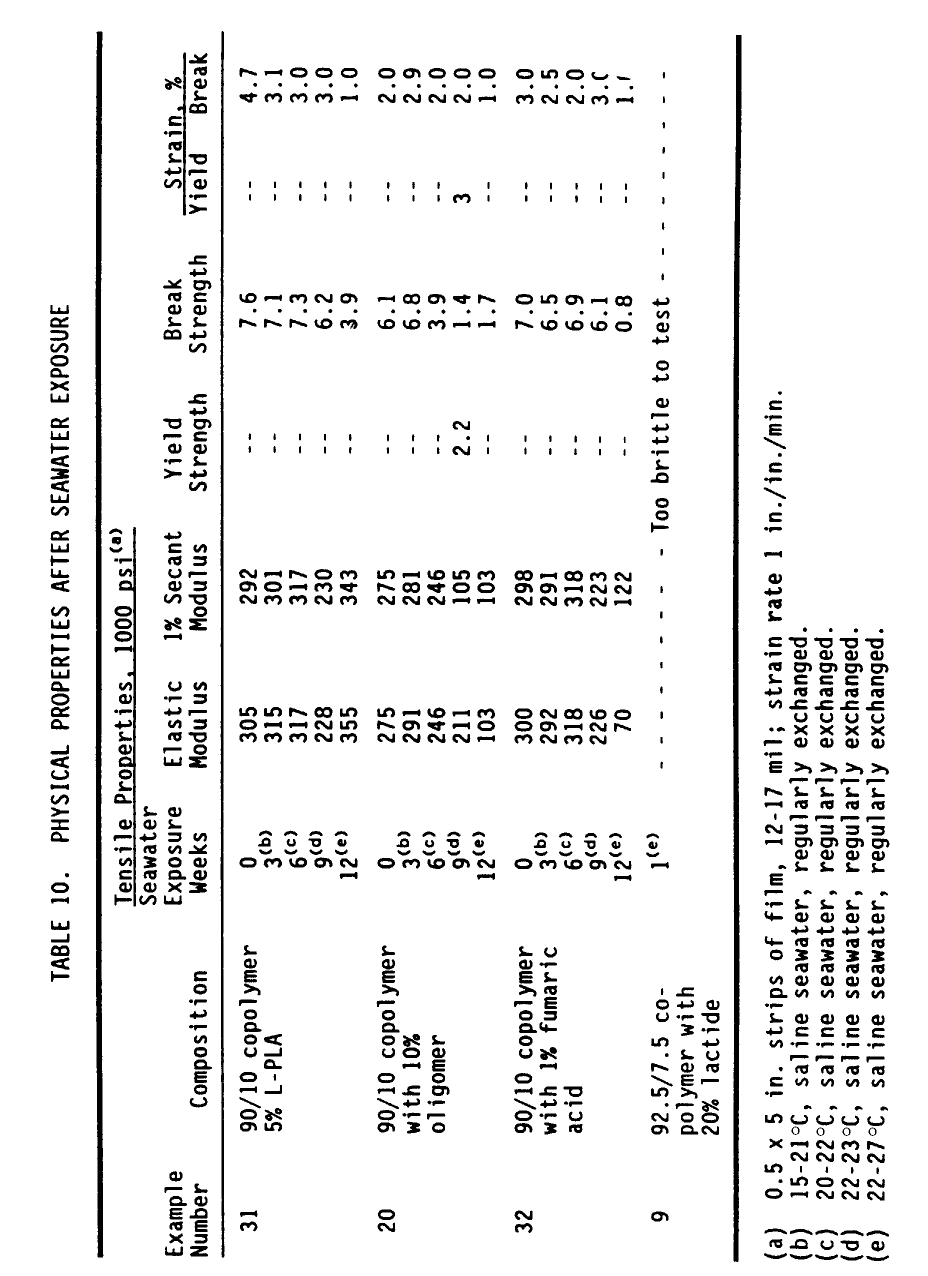

The above examples establish that an all lactic acid composition can be a pliable thermoplastic useful for flexible, plastic containers. By way of comparison, nonplasticized homopoly (L-lactide) is a highly crystalline polymer with a tensile strength of about 7000 psi with an elongation of 1 percent and an initial modulus of 500,000 psi. It is very brittle, opaque, and crazes easily. It is not a well behaved thermoplastic, nor is it transparent. Poly (racemic D,L-lactide) is an amorphous, glassy, polymer with a glass transition temperature of approximately 50 C, a tensile strength of about 6300 psi, an elongation of approximately 12 percent, and an initial modulus of 160,000 psi. It is also very brittle although transparent. In stark contrast, a polymer of L-lactide/racemic D,L-lactide copolymer that is plasticized with lactide monomer is remarkably different. For example, the plasticized polymers can have a tensile strength of approximately 3900 psi, an elongation of 431 percent, and an initial modulus of 56,000 psi. The plasticized polymer is clear and colorless and the blend must be heated to above 100°C to remove the plasticizer.

Although theory would predict a more amorphous structure as a result of plasticization, what is surprising is the pliable, transparent, stable compositions that can arise, and, secondly, the nearly exact fit of properties needed for certain packaging applications, such as polyethylene. This invention comes at a time when there is a need for such initial properties in a material that is slowly environmentally biodegradable since it could alleviate plastic pollution problems.

It will be apparent to those skilled in the art that extremely intimate blends of high polymers and plasticizers are a rarity. Plasticization allows a wide latitude in the initial physical properties and the time for environmental biodegradation.

The amount of plasticizer in the polymer depends on the compositional characteristics desired. If lactide is used as plasticizer the range is preferably 5 to 40 weight percent whereas if only oligomers of lactide or lactic acid are used the range may be from 2 to 60 weight percent. Surprisingly, oligomer may be added at up to 30 weight percent without substantially affecting the tensile strength or modulus. See Figures 3 and 4. Addition of 30 to 60 weight percent oligomers produces significant plasticization and attenuation of physical properties. This adds great economy to the composition since oligomeric lactic acid is cheaper than high polymer lactic acid. Oligomer may be prepared from lactic acid or any lactide. It is important to note that the oligomer of lactic acid normally contains significant amounts of lactic acid unless removed. This is an important consideration in tailoring compositions having specific properties. Those skilled in the art and knowing the teachings of this invention will be able to select reaction conditions to obtain appropriate chain lengths for the polymer, and the proportions of polymer and plasticizer so as to obtain fabricated compositions having physical properties similar to commonly used packaging thermoplastics and yet degrade comparatively rapidly. For example, higher amounts of plasticizer result in polymers having increased flexibility and increasingly tough physical properties, however, an increasing degradation rate will also be obtained. Further, shorter chain lengths for the polymer will require less plasticizer to obtain the same properties as with longer lengths.

Further provided by the invention is a process for producing a environmentally biodegradable composition that is a plasticized polymer of polylactic acid having the formula (I). The process comprises preparing one or more lactide monomers and catalyst; polymerizing the monomers to form a polymer at a temperature sufficiently low to allow the polymerization reaction to be stopped prior to complete polymerization; monitoring the level of monomer to determine the amount of remaining monomer; and stopping the reaction prior to complete polymerization at a determined amount of monomer so that unreacted monomer of a predetermined amount is trapped in association with the polymer. The lactide monomers of the process may be selected from D-lactide, L-lactide, meso D,L-lactide, racemic D,L-lactide, and mixtures thereof, and incorporating additional plasticizer into the polymer whereby the plasticizer is further selected from D-lactide, racemic D,L-lactide, meso D,L-lactide, lactic acid, oligomers of lactic acid, oligomers of lactide, and mixtures thereof. Preferably polymerization of the monomers is at a temperature less than 129°C. Further processing of the plasticized polymer into a final product is preferably at a temperature sufficiently low to retain the plasticizer in the polymer. This temperature may be above 129°C. If additional monomer and/or oligomer are added the retention of monomer is of course not as critical.

Further provided by the invention is a process for producing a polymer of formula I comprising preparing one or more lactide monomers and catalyst; polymerizing the monomers to form a polymer; and incorporating plasticizer in a separate step into the polymer whereby the plasticizer is selected from D-lactide, L-lactide, D,L-lactide, oligomers of lactic acid, and mixtures thereof.

The compositions prepared in accordance with the invention should have a tensile strength of 300 to 20,000 psi, an elongation to failure of 50 to 1,000 percent and a tangent modulus of 20,000 to 250,000 psi. Preferably for a polyolefin replacement the compositions have a tensile strength of at least 3000 psi, an elongation to failure of at least 250 percent, and a tangent modulus of at least 50,000 psi.

The homopolymers and copolymers prepared in accordance with the present invention are insoluble in water but upon constant contact with water are slowly degradable. However, degradation is fast when compared to polyolefin compositions that are replaced by the invention. Thus, throwaway objects made from the polymers are environmentally attractive in that they slowly degrade to harmless substances. If objects made from polymers of the invention are incinerated, they burn with a clean, blue flame.

Yet further provided by the invention is a method for replacing a thermoplastic composition with the biodegradable composition prepared in accordance with the invention where the thermoplastic composition comprises first orientable polymer units, by replacing the first polymer units with a second orientable polymer having an unoriented tensile strength of 300 to 20,000 psi, an elongation to failure of 50 to 1,000 percent, and a tangent modulus of 20,000 to 250,000 psi; wherein the second polymer comprises polylactic acid units of the structure in formula I, wherein n is the number of repeating units and n is an integer, 150 ≤ n ≤ 20,000 and is plasticized with a plasticizer selected from lactide, oligomers of lactic acid, oligomers of lactide and mixtures thereof. The method is useful for polyolefin compositions and particularly polyethylene and polypropylene as well as polyvinyl chlorides and polyethylene terephthalate. In addition to the above list the method is useful for replacement of polymers of styrene, vinyl acetate, alkyl methacrylate, alkyl acrylate. It is understood that copolymers made from mixtures of the monomers in the listed group and physical mixtures of the polymers and copolymers of the above group are likewise replaceable.

The environmentally biodegradable compositions disclosed herein are completely degradable to environmentally acceptable and compatible materials. The intermediate products of the degradation: lactic acid is a widely distributed naturally occurring substance that is easily metabolized by a wide variety of organisms. Its natural end degradation products are carbon dioxide and water. Contemplated equivalents of these compositions such as those that contain minor amounts of other materials, fillers, or extenders can also be completely environmentally degradable by proper choice of materials. The compositions herein provide environmentally acceptable materials because their physical deterioration and degradation is much more rapid and complete than the conventional nondegradable plastics that they replace. Further, since all or a major portion of the composition will be polylactic acid, and/or a lactic acid derived lactide or oligomer, no residue or only a small portion of more slowly degrading residue will remain. This residue will have a higher surface area than the bulk product and an expected faster degradation rate.

The preferred composition prepared in accordance with the present invention comprises polymerized lactic acid units with the repeating unit of formula I, wherein n is an integer with a value between 75 and 10.000 and the alpha carbon is a random mixture of D and L (or R and S) with a preponderance of one of the pure enantiomeres. When n is low, the polylactic acid, PLA, is easily processible, but is considerably weaker than when n is larger. When n is quite large, e.g., 7000 or greater, the PLA is quite strong but difficult to injection mold. Preferably n is approximately 500 to 3000 for the best balance of melt-processibility and end-use physical properties. The monomers are selected in L (or D)/D,L-ratios of polymerized lactic acid or their cyclic dimer, lactide, as further discussed below. Both lactic acid and lactide achieve the repeating PLA unit, shown above, but lactide is preferred since it more easily obtains the higher molecular weights necessary for good physical properties. Since lactide, which has the structure: 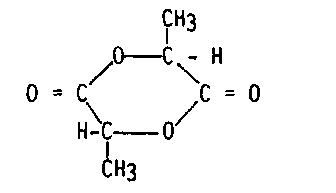 has two alpha carbons which are assymetric, there are three types of lactide, viz., D,D- (or D-); L, L- (or L-); and meso D,L-lactide.

D-lactide is a dilactide, or cyclic dimer, of D-lactic acid. Similarly, L-lactide is a cyclic dimer of L-lactic acid. Meso D,L-lactide is a cyclic dimer of D- and L-lactic acid. Racemic D,L-lactide comprises a 50/50 mixture of D-, and L-lactide. When used alone herein, the term "D,L-lactide" is intended to include meso D,L-lactide or racemic D,L lactide. The term dispersed as used herein means the material is homogeneously and intimately mixed with the polymer.

Pure L-PLA has poor processing characteristics, easily crazes and becomes opaque. Pure D,L-PLA processes easily but is not sufficiently rigid to be an adequate OPS offset. The copolymer ratio of between 85/15 to 95/5, and preferably 90/10, L-lactide/D,L-lactide is a preferred embodiment of the invention. At higher ratios than 95/5, the copolymer is difficult to thermoform without crazing and easily becomes opaque at room temperature. At lower ratios than 85/15, the lactide copolymers exhibit lower than desirable moduli for OPS offsets. In between these limits the copolymers are quenched from the melt in typical manufacturing/processing equipment of plastics technology to achieve films and moldings which are clear, colorless, and extremely rigid. Their properties as formed, above, are closely matched to those properties of an OPS.

Another advantage of this invention is that the all-lactic acid copolymer can utilize inexpensive feedstocks. Corn syrup via starch and corn can be fermented to either L- or racemic D,L-lactic acid, depending on the microorganism. Racemic D,L-lactic acid is cheaply obtainable via ethylene which can be oxidized to acetaldehyde, which is reacted with hydrogen cyanide to form lactonitrile, which is hydrolyzed to racemic D,L-lactic acid. Lactide is simply obtained by distillation of lactic acid. No change of the stereochemistry of the asymmetric carbon occurs in transforming lactic acid to lactide by ordinary distillation/condensation methods.

While the reaction of L-lactide and D,L-lactide is discussed herein, it is to be understood that the reactions specifying L-lactide may also use D-lactide. Thus the reaction of D-lactide and D,L-lactide according to the method described herein will give an equivalent product; the only difference being that it rotates light in a different direction.

The copolymers of the present invention are preferably formed by heating the mixture of monomers to form a homogeneous melt and adding a catalyst to cause the lactides to undergo a ring-opening polymerization. The polymerization is preferably carried out in an inert, anhydrous, atmosphere, such as nitrogen or argon, or in a vacuum. Suitable catalysts include divalent metal oxides and organo-metallic compounds such as stannous octoate, zinc acetate, cadmium acetate, aluminum acetate or butanoate, tin chloride, tin benzoate, and antimony oxide. Stannous octanote is the preferred catalyst because of its high solubility in the monomers, ease of preparation in anhydrous form, and low toxicity. The amount of catalyst required can vary from approximately 0.02 to 2 percent by weight, based on monomers and is preferably about 0.2 percent. The molecular weight and melt viscosities of the copolymers are controllable by the amount of catalyst and/or chain-transfer agents such as glycolic acid. The reaction temperature of the polymerization is between approximately 100 to 200° C. The least color formation occurs below 140 C and the rate of polymerization is best above 135 C. Since racemic D,L-lactide melts at 127°C it is best for conversion of monomer to polymer to polymerize at a temperature above 127°C.

Where clarity and transparency is required, as with OPS offsets, the copolymers of this invention are polymerized in an inert atmosphere above their melting points, which are generally in the 125 to 150°C range. The molten lactide copolymer can be extruded from the polymerizer in strands and rods, quenched, pelletized and stored in bags for use in subsequent molding and extrusion operations.

Similarly, clarity of thermoformed packaging films and shaped articles is achieved by molding and extruding above the copolymer's melting points and fast cooling the fabricated item. Thereafter, the copolymers remain transparent unless heated for several hours above their glass transition temperature, Tg, and below the melting point, Tm. Slow cooling of thermoformed sheets, slabs, films, and molded items can induce spherulite crystallinity in the copolymers which gains improvement in the heat stability of the fabricated item, but causes some loss of transparency. Nucleating agents such as sodium benzoate, calcium lactate, and the like, can also induce rapid and substantial crystallinity. A modest amount of drawing of the copolymer, between its Tg and Tm, induces orientation of the polymer molecules and can substantially improve physical properties without loss of transparency.

Blending of different types of lactide polymer or copolymer can substantially change the physical properties. As an example, the melt-blending of the high-melting L-lactide polymer with a lower melting lactide copolymer can provide a transparent material which has a sufficient amount and type of crystallinity to remain transparent. Those skilled in the art will recognize that transparency in molded films, great stiffness, elevated heat distortion temperature, thermo-processibility, and environmental biodegradability are a rare combination of properties. Thus, the polymers can be blended, as well as nucleated, oriented, and controlled by molecular weight to provide a great deal of latitude in the processibility and final properties in the final compounded thermoplastic.

The copolymers of the present invention will hydrolyze back to lactic acid in the presence of moisture. In the presence of ambient air and humidity the hydrolysis becomes evident in about 12 to 18 months time. The copolymers then become sticky, somewhat opaque, and very brittle. When immersed in water the copolymers show obvious hydrolysis effects in 1 to 4 months time, depending on the composition, molecular weights, the ambient temperature, their surface-to-volume ratio, and the particular, aqueous environment the copolymers are placed in. Microorganisms can further reduce the lactic acid to carbon dioxide and water. As an approximate measure, the copolymers have a shelf life of several months, but disappear within about a year when thoroughly wet.

The following examples are merely illustrative of the present invention. In Examples 1B to 7B, a composition series was prepared and evaluated. It was discovered, in contrast to the prior art, that there are distinct differences in the processing behavior and physical properties of the L-lactide/D,L-lactide copolymers.

### Example 1B

In a dry, 500 ml, round-bottom flask was charged 160 g of L-lactide (Purac, Inc., "triple-star" grade) and 40 g of racemic 0,L-lactide (Purac, Inc., "triple star" grade). This mixture was heated for approximately 1 hour at 123-129°C under a stopper with a continuous nitrogen purge through a stopper inlet and outlet. The monomers form a clear melt, which is mixed thoroughly by swirling the melt. Catalyst solution was prepared and dried by azeotropic distillation, that is, 10 ml of stannous octoate (Polysciences, Inc.) was dissolved in 60 ml of toluene; 10 ml of toluene, with trace water, was distilled to a Dean-Stark trap that was vented via a drying tube. A 0.20 ml quantity of the stannous octoate solution was pipetted into the melt and mixed thoroughly. The nitrogen sweep continues and the melt becomes increasingly viscous over the next 3 hours. Heating continues at 123-127 °C for 20-24 hours. The mixture was allowed to cool to room temperature and the flask cooled further with liquid nitrogen behind a shield. The glass shatters and is removed from the polymer by tapping. The copolymer is clear and colorless and is evaluated in a series of tests shown in Table 1B. Films were compression molded at 170°C in a heated hydraulic press for later tensile testing. Slabs, 1/8 inch thick were molded for impact testing by notched Izod, ASTM, D256 and heat deflection temperature, ASTM, D648. Glass transition temperature (Tg) and melting point (Tm, center of the endotherm) were evaluated by differential scanning calorimetry (DSC).

### Examples 2B-7B

The procedures of Example 1B were repeated except that the ratio of L- and racemic D,L-lactide were changed as shown in Table 1B with the test results. The pure L-lactide polymer, Example 7B, would not always mold well at 170 - 200°C since it frequently crazed badly on cooling in the mold. Frequently, on cooling, it opacified.

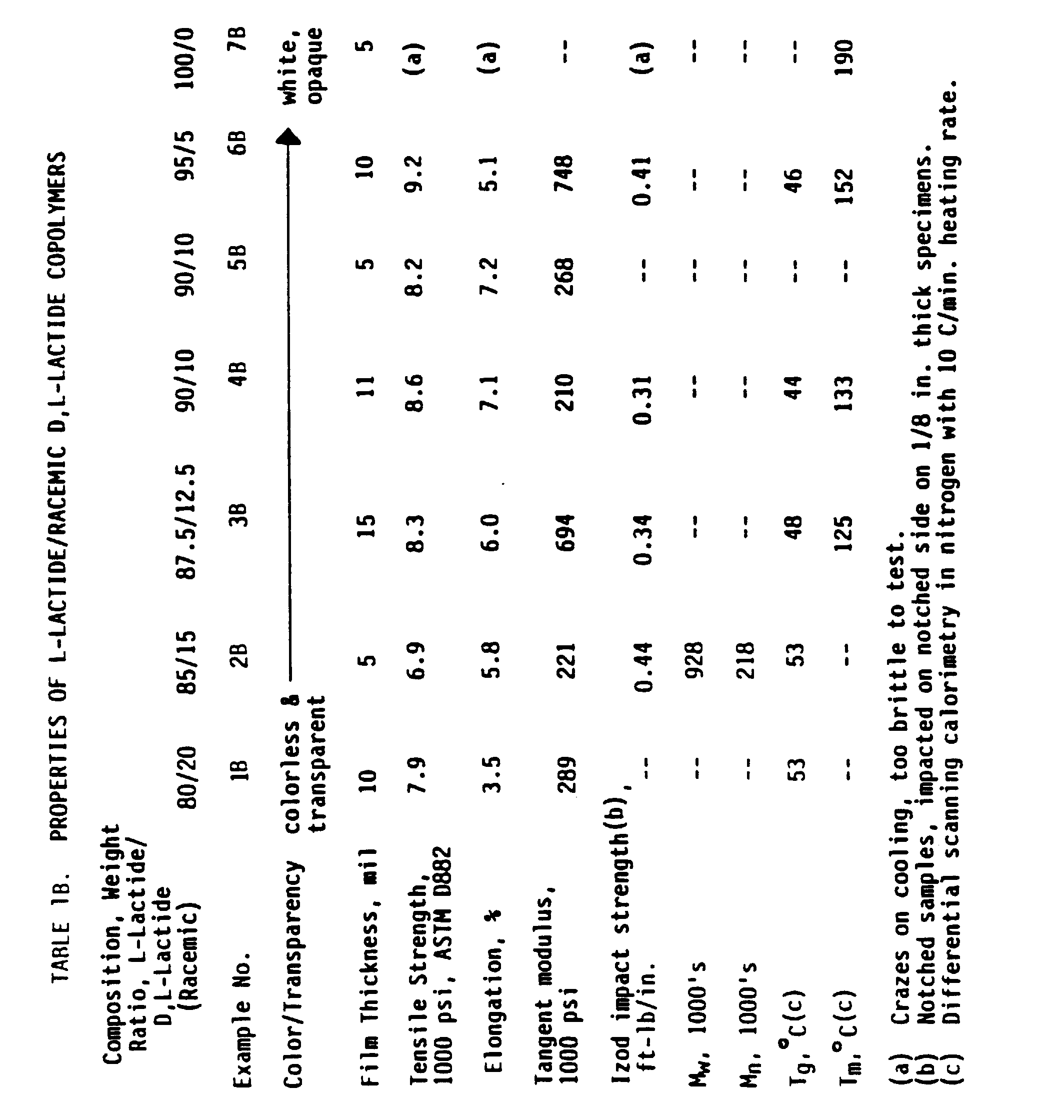

### Example 8B

Similar to Examples 4B and 5B, a 90/10 weight ratio copolymer of L-lactide/racemic D,L-lactide was prepared. Into a dry, nitrogen-swept, 2-liter flask was placed 1045.8 g L-lactide and 116.4 g of racemic D,L-lactide. A 1.0 ml quantity of anhydrous stannous octoate (0.2 ml per ml of toluene) solution was added. The flask was swept with nitrogen overnight, then heated in a 141°C oil bath until the monomers are melted and well mixed, and the heating decreased slowly to 125°C and continued for 72 hours. The polymer slowly whitens on cooling. After removing the glass, the cloudy, colorless, glassy copolymer was evaluated. Gel permeation chromatography obtains a weight-average molecular weight (M_{w}) of 522,000, and a number-average molecular weight (Mₙ) of 149,000.

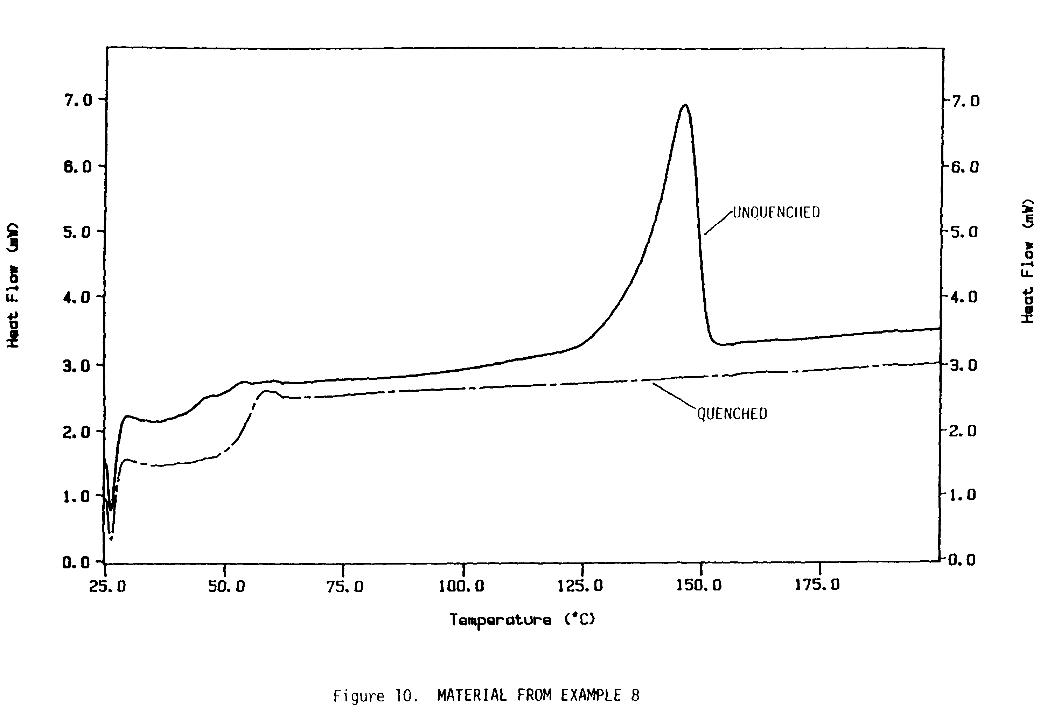

A DSC of the lactide polymer reveals a strong Tm at 145°C, see Figure 10. The lactide polymer was melted, quenched, and examined again by DSC to reveal no crystallization or melting points. However, a Tg appears at approximately 50-55°C. The results show the polymer can be crystalline or amorphous, depending on its heat history.

### Examples 9B - 12B

The composition series was extended, using the procedures of Example 1B except other L- and racemic D,L-lactide ratios were used and heating was 2 hours 125°C, 14 hours 125-147°C, then 2 hours 147-131°C. The results are shown in Table 2B.

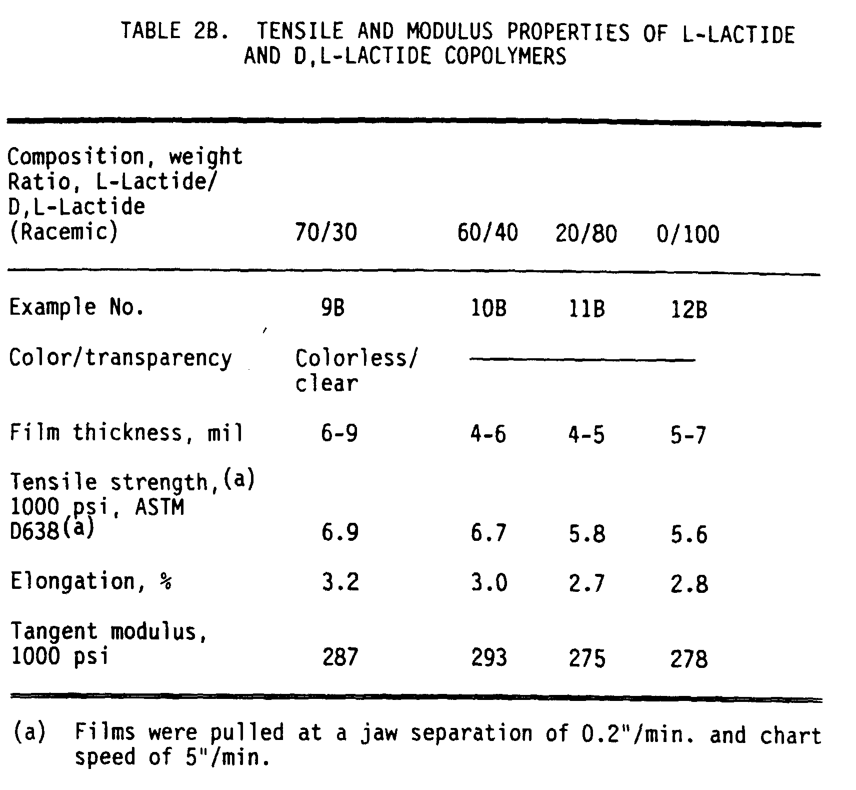

The results of the above examples reveal that only certain compositions have the required properties for an OPS offset. The main requirements for an OPS-like material are clarity and colorlessness, tensile strength greater than 7000 psi, tangent modulus (a measure of stiffness) greater than 400,000 psi and well-behaved thermoplasticity. Table 3B lists some side-by-side comparisons of a crystal polystyrene (OPS) and a 87.5 weight percent L-lactide and 12.5 weight percent racemic D,L-lactide random copolymer.

**TABLE 3B.**

| PHYSICAL PROPERTY COMPARISONS | | |
|---|---|---|
| Property | Poly(lactic acid), Example 3B | Crystal Polystyrene |
| Impact strength, notched Izod, ft-lb/in. | 0.4 | 0.4 |
| | | |
| Ultimate tensile strength, psi | 8300 | 7400 |
| | | |
| Elongation, % | 6.0 | 4.0 |
| | | |
| Elastic modulus, psi | 694,000 | 450,000 |
| | | |
| Deflection temperature, °F under load, 264 psi | ^{(a)} | 200 |
| | | |
| Specific gravity | 1.25 | 1.05 |
| | | |
| Rockwell hardness | ^{(b)} | M75 |
| | | |
| Vicat softening point, °F | ^{(c)} | 225 |
| | | |
| Melt flow rate, D1238(G) | 40-46^{(d)} | 1.7 g/10 min.^{(e)} 1.6 g/10 min.^{(f)} |

| | | |
|---|---|---|
| (a) Depends on heat history. | | |
| (b) Shore D = 97. | | |
| (c) Shore Tₘ = 125°C (257°F) at 10 degree/min. | | |
| (d) Flow rate decreases at lower temperature. | | |
| (e) Listed by manufacturer. | | |
| (f) By our experiment. | | |

### Example 13B

The copolymer of Example 2B was molded and remolded several times to determine if color would develop in the films and the molecular weights remained high. This determines if the copolymer can be recycled, an important consideration for manufacturing practices. The results of Table 4B show that the copolymer remained completely transparent and colorless after repeated heating and molding despite the fact that the copolymer was repeatedly exposed to air at elevated temperatures.

**TABLE 4B.**

| EFFECT OF MOLDING ON LACTIDE COPOLYMER | | | | | |
|---|---|---|---|---|---|
| Example No. | History | Appearance | M_{w}, 1000's | Mₙ, 1000's | M_{w}/Mₙ |
| Example 2B^{(a)} | Not molded, directly from polymerization | Completely transparent and colorless | 928 | 218 | -- |
| | | | | | |
| Example 13B^{(a)} | Ex. 2B after molding^{(b)} | " | 301 | 135 | 2.22 |
| | | | | | |
| Example 13B^{(a)} | Ex. 2B after molding 6 times^{(b)} | " | 137 | 56.7 | 2.42 |

| | | | | | |
|---|---|---|---|---|---|
| (a) 85/15, L-lactide/racemic D,L-lactide copolymer. | | | | | |
| (b) Compression molding at 167°C (333°F) for 7 minutes to 5-mil film. | | | | | |

### Examples 14B-18B

The copolymers of Examples 2B, 3B and 6B were compression molded into films of approximately 20 to 30-mil thickness and were placed in a heated Instron tester where the films were drawn 5 times their length at 83°C at a rate of 0.5 inch per minute. The films were cooled quickly upon removal from the Instron, and found to be approximately 5-mil in thickness. They were clear and colorless. Tensile properties were evaluated and are listed in Table 5B. When drawn 8 to 10 times their length, the films show evidence of crystallinity formation by virtue of haze development and some loss of transparency.

The results demonstrate that very thin films can be made with adequate stiffness and transparency for an OPS offset. Thus, despite the higher density of the lactide copolymers compared to polystyrene, less material can be used for stiff OPS offsets.

**TABLE 5B.**

| PROPERTIES OF L-LACTIDE/RACEMIC D,L-LACTIDE COPOLYMERS AFTER ORIENTATION^{(a)} | | | | | |
|---|---|---|---|---|---|
| Composition, weight Ratio, L-Lactide/D, L-Lactide (Racemic) | 85/15 | 85/15 | 85/15 | 87.5/12.5 | 95/5 |
| Example Number | 14B | 15B | 16B | 17B | 18B |
| | | | | | |
| Film thickness, mil | 5.5 | 5.0 | 6.5 | 5.0 | 4.0 |
| | | | | | |
| Tensile strength, 1000 psi | 14.0 | 14.7 | 15.0 | 13.0 | 16.0 |
| | | | | | |
| Elongation, % | 31.5 | 15.4 | 30.0 | 23.8 | 37.4 |
| | | | | | |
| Tangent modulus, 1000 psi | -- | 564 | 419 | 432 | 513 |

| | | | | | |
|---|---|---|---|---|---|
| (a) 5X oriented at 83°C using a draw down speed of 0.5 in./min. on Instron. | | | | | |

### Example 19B

Films of the copolymers of lactide of Table 1B were immersed in water for several months interval. The copolymers remained clear for approximately 2 months; after 3 months a slight haziness developed. Upon setting on the shelf in humid air and with frequent handling, the films remain virtually unchanged for approximately 1 year although Instron data will show a slow decrease in the strength and elongation after several months. In a landfill, the buried films disappear in 6 months to 2 years, depending on the moisture, pH, temperature, composition, surface-to-volume ratio, and biological activity of the landfill. All of the films burn with a clean, blue flame.

### Example 20B

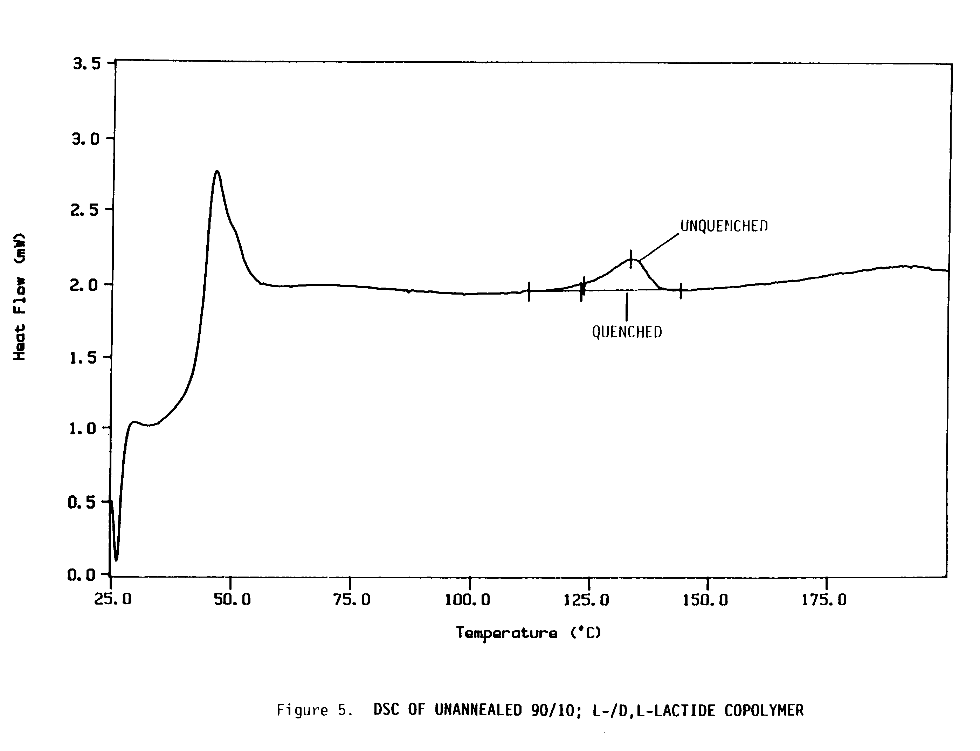

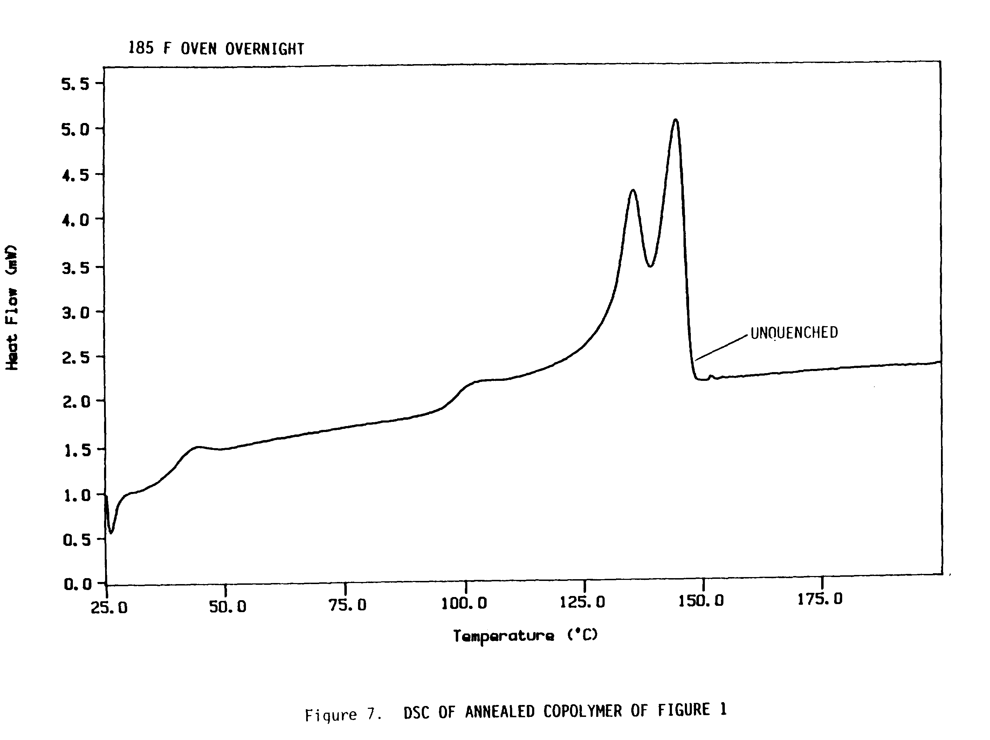

The lactide copolymer of Example 5B (quenched, compression-molded film) was examined by DSC and found to have less than 2 percent crystallinity, see Figure 5, in the vicinity of 130°C. A 1/8 inch thick sample of the copolymer of Example 5B was annealed in a 185°F oven for 16 hours. The sample turned hazy and the DSC of the sample, see Figure 7 revealed a pronounced increase in the crystallinity. The sample showed a 264 psi heat deflection temperature (HDT) of 90° to 95° C. A similar sample without annealing exhibited a heat deflection temperature of 50° to 55°C, which corresponds to its Tg.

### Example 21B

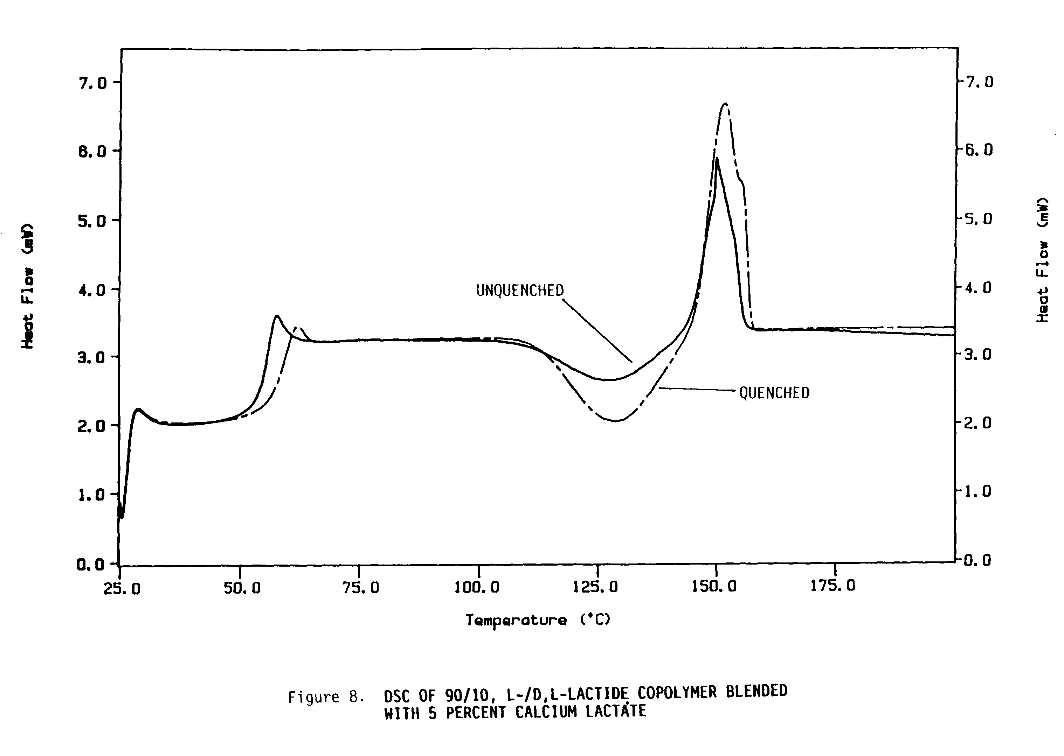

Calcium lactate, 5 weight percent, was blended on a heated mill roll with the lactide copolymer of Example 5B at 170 °C for approximately 5 minutes. The blend was stripped off the roll as a sheet and examined. It was stiff, strong, and hazy. Optical microscopy at 82X reveals heterogeneous domains in the size range of from a few microns to 30 microns. DSC reveals a substantial increase in crystallinity in the vicinity of 145°C, see Figure 8, which remain on quenching and reheating. The results, above, comparing Examples 8B, 20B, and 21B, show that nucleating agents are more prompt and efficient in inducing crystallinity in lactide copolymers. Nucleating agents such as salts of carboxylic acids may be used, salts of lactic acid are preferred.

### Example 22B

In a 500-ml, 3-neck, round bottom flask, equipped with a mechanical stirrer and a nitrogen inlet and outlet, was placed 180.7 g of L-lactide and 40.2 g of racemic D,L-lactide (both Boehringer and Ingelheim, grade S). The contents of the flask were heated to 110°C under a nitrogen sweep to melt the lactides and 20.1 g of polystyrene (Amoco R3, melt index 3.5 g/10 min.) was added. The polystyrene swelled highly and partially dissolved with stirring overnight while advancing the heat to 185°C. The temperature was decreased to 141°C and 0.2 ml of anhydrous stannous octoate solution (0.2 ml/ml of toluene) was added. The stirrer was turned off and the lactides allowed to polymerize at 141°C over 3 days time. The highly swollen, polystyrene floats to the top after turning off the stirrer. The lower, polylactide phase was cooled and examined by DSC. The sample has a low Tg, approximately 35°C, and is otherwise lacking in apparent temperature transitions. Compression-molded films are clear, colorless, and very pliable. These results indicate that the polystyrene thoroughly interrupts crystallinity formation.

### Example 23B

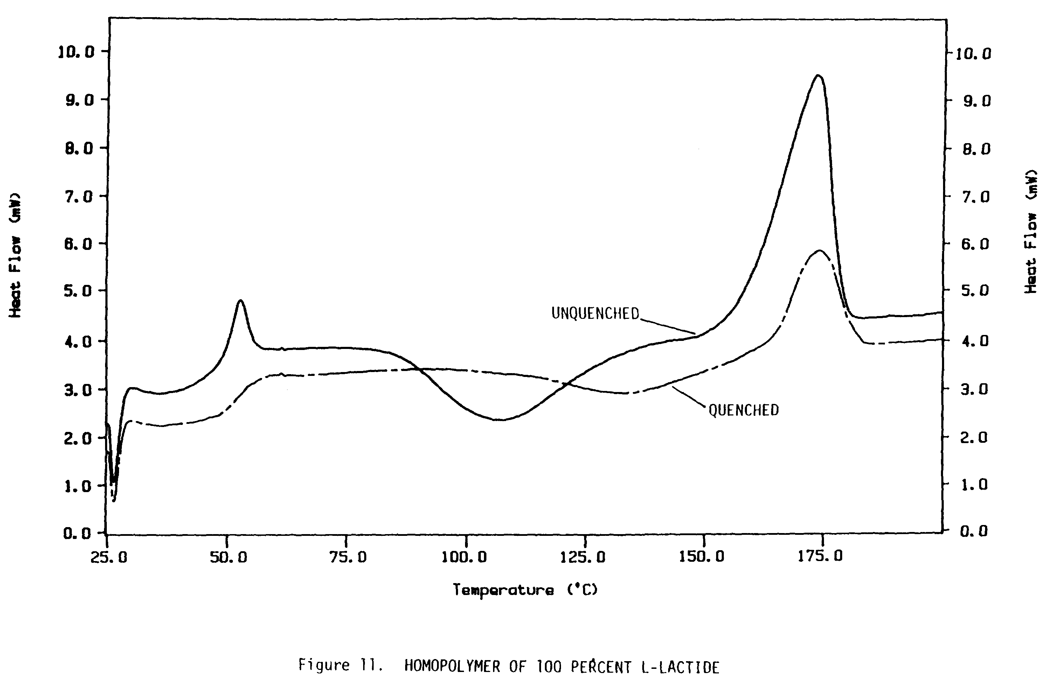

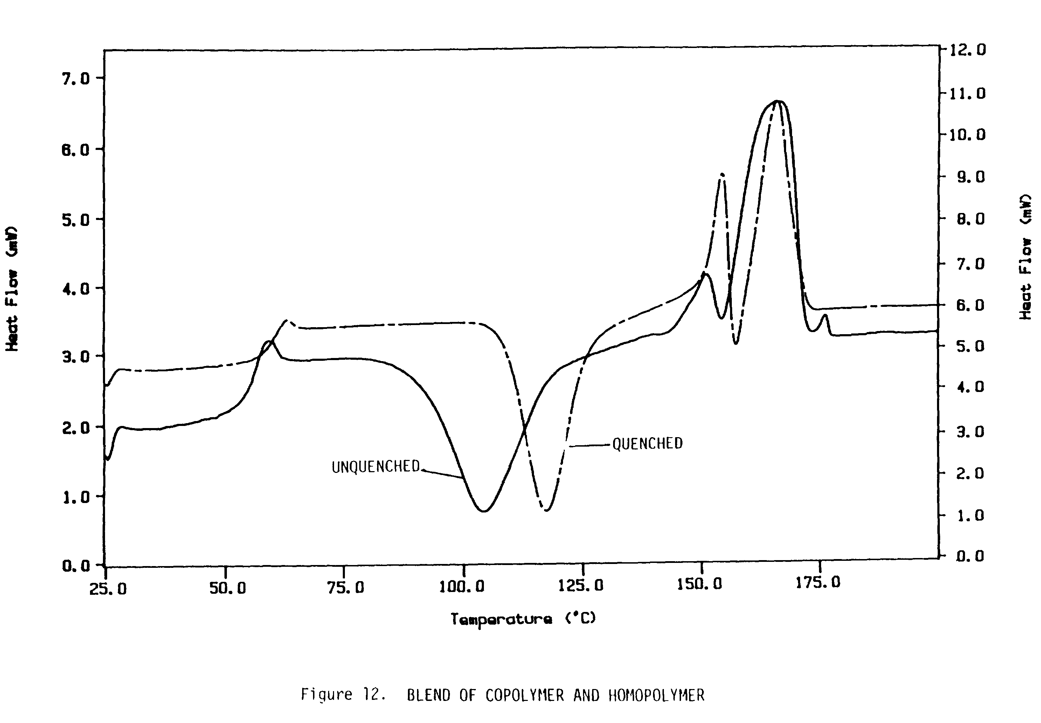

The lactide copolymer of Example 8B was mill-roll blended with 20 weight percent of the homopolymer of L-lactide produced in Example 7B. A sample of the homopolymer was analyzed by DSC, see Figure 11. The blended sample was examined by DSC and found to have a Tg of 59-63°C and strong Tm's at 150°and 166°C, see Figure 12. Films were clear to slightly hazy, depending on their cooling rate after pressing. Quenched samples easily crystallize on heating to approximately 80°- 90° C. As a result the heat deflection temperature of the blend is now quite high. The blend becomes hazy at 80-90°C but does not deflect with heat as does the unblended 90/10 copolymer. Tensile data as shown in Table 6B were obtained on unoriented, compression-molded films and compared to similarly obtained data for polystyrene.

**TABLE 6B.**

| COMPARISON OF BLEND OF POLYLACTIDE OF EXAMPLE 23B WITH CRYSTAL POLYSTYRENE | | |
|---|---|---|
| | Example 23B^{(a)} | Crystal Polystyrene^{(a,b)} |
| Film thickness, mil | 8 | 14 |
| | | |
| Tensile strength, ASTM D882, 1000's psi | 7.7 | 6.0 |
| | | |
| Elongation, %, to yield | 6.5 | 3.2 |
| | | |
| Tangent modulus, 1000,'s psi | 323 | 267 |

| | | |
|---|---|---|
| (a) Thin films, unoriented, compression-molded specimens | | |
| (b) Melt Index 1.7 | | |

This example illustrates that melt blending is an excellent way to improve the properties of the copolymer so that advantageous properties similar to polystyrene are realized. The higher the amount of homopolymer based on L-lactide (or D-lactide) blended with the polymer the higher will be the heat deflection temperature, however, haziness will also increase. Thus addition of homopolymer may be combined with other methods of increasing polystyrene like properties while still retaining clarity

As a further example orienting films produced from the polymer increases the tensile properties. At eight to ten times the draw the physical properties are still increasing but the material becomes hazy. The degree of orientation will thus need to be controlled and combined with the other property changing methods to achieve optimum polystyrene like characteristics.

### Examples 24B-27B

Examples 24B to 27B were polymerizations of lactide with controlled amounts of chain transfer agents, demonstrating that molecular weights can be controlled using transfer agents such as glycolic acid. The results are shown in Table 7B. A nearly straight line relationship exists between the amount of transfer agent and the reciprocal of the weight average molecular weight. A preferred chain transfer agent is lactic acid.

**TABLE 7B.**

| MOLECULAR WEIGHT CONTROL USING CHAIN TRANSFER AGENTS | | | | |
|---|---|---|---|---|
| Example No. | PPH of^{(a)} CTA | Mₙ^{(b)} | M_{w}^{(b)} | M_{w}/Mₙ |
| 24B | 0.22 | 13,500 | 107,300 | 8.0 |
| | | | | |
| 25B | 0.45 | 12,800 | 66,700 | 5.2 |
| | | | | |
| 26B | 0.90 | 7,300 | 29,900 | 4.1 |
| | | | | |
| 27B | 1.80 | 4,700 | 13,900 | 2.9 |

| | | | | |
|---|---|---|---|---|
| (a) Parts of glycolic acid chain transfer agent (CTA) per hundred parts of lactide in polymerization recipe. | | | | |
| (b) Gel permeation chromatography in tetrahydrofuran solvent, 23 C, with 10⁶, 10⁵, 10⁴, and 10³ anhstrom columns, number average, Mₙ, and weight average, M_{w}, molecular weights are calculated compared to monodisperse polystyrene standards. | | | | |

### Example 28B

A 4.0 mil, compression-molded film of the lactide copolymer of Example 2B was evaluated as a barrier film by ASTM methods. The results are shown in Table 8B. The lactide copolymer is a much better barrier to carbon dioxide and oxygen than is polystyrene. By comparison to some other polymer barrier films, the lactide copolymer is an adequate barrier film for many packaging applications.

**TABLE 8B.**

| EXAMPLE 28B PERMEABILITY TO GASES^{(a)} | | | | |
|---|---|---|---|---|
| Units | Lactide Copolymer, Example 28 | Crystal^{(b)} Polystyrene | Terephthalate | Vinyldiene^{(b)} Chloride-vinyl Chloride Copolymer |
| cc/100 sq. in./ 24 hr/atmos | | | | |
| | | | | |
| CO₂ | 32.1 | 900 | 15-25 | 3.8-44 |
| | | | | |
| O₂ | 19.9 | 350 | 6-8 | 0.8-6.9 |

| | | | | |
|---|---|---|---|---|
| (a) ASTM D1434-75, Example 2B was a 4.0 mil, compression-molded film. | | | | |
| (b) Values from Modern Plastics Encyclopedia. | | | | |

### Example 29B

Sheets, 1/8 inch thick of the lactide copolymers of Examples 1B-6B were immersed overnight in a mixture of petroleum ether and methylene chloride. At ratios of 70/30 to 60/40, petroleum ether/methylene chloride, the copolymers would foam when placed in boiling water. Irregular, but well expanded, foams would form.

### Example 30B

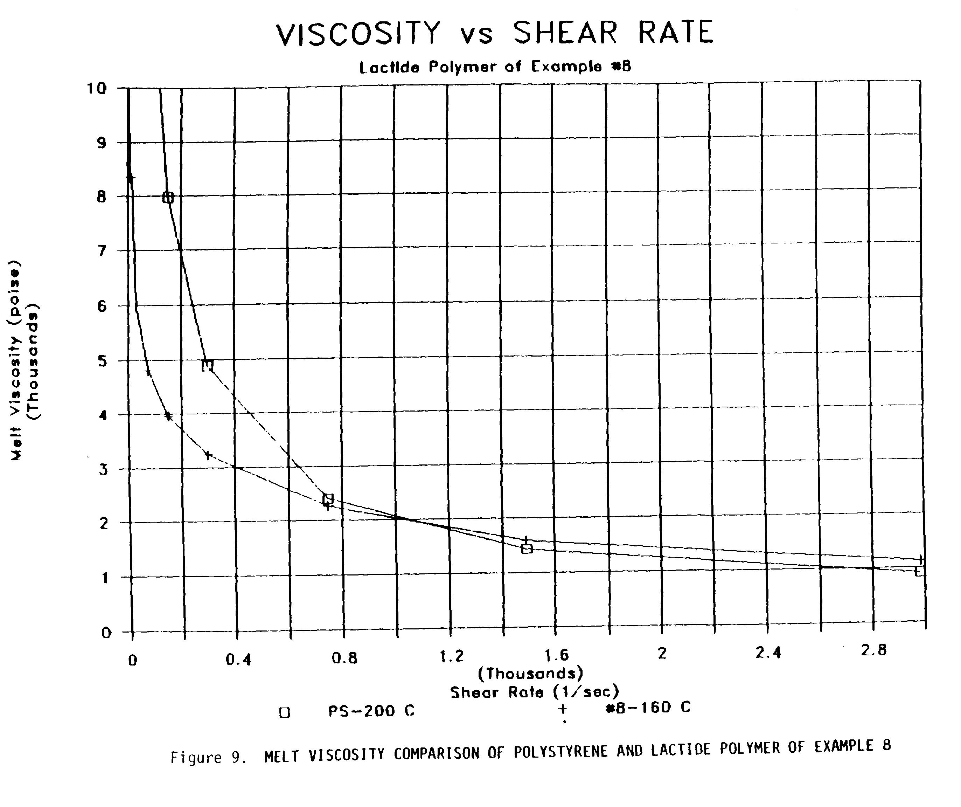

A comparison was made of the melt viscosities of a commercial, crystal polystyrene (Type 201, Huntsman Chemical Corp.) and the lactide polymer of Example 8B. The melt index, ASTM D1238 (G), of the polystyrene was 1.6 g/10 min. at 200°C using the standard 5 Kg weight. The melt index of the lactide polymer was 40-46 g/10 min. under the same conditions, however, at 160°C the value was 8.0 g/10 min. A more detailed comparison of melt viscosities was obtained by observing the melt viscosities of the two polymers in an Instron Capillary Viscometer. The comparative results are shown in Figure 9. The shear rates normally encountered during extrusion and injection molding are approximately 100 to 1000 reciprocal seconds. Inspection of the data of Figure 9 shows that the melt viscosity of the lactide polymer at 160° C is very similar to that of the polystyrene at 200°C.

The above results illustrate that lactide polymers can be melt-processed, at lower temperatures than polystyrene, by very similar methods.

### Examples 31B-34B

Small, test polymerizations of purified (recrystallized and dried) mesolactide (meso D,L-lactide) were carried out as the homopolymer and the copolymer. The molecular weights were evaluated by GPC and compared to analogues of D,L-lactide. The results are presented in Table 9B. The polymers were melt pressed into films and their physical properties evaluated and compared as shown in Table 10B. Within experimental differences of sheet thickness and molecular weight, the copolymers are similar within experimental error. The homopolymer of mesolactide is somewhat weaker.

**TABLE 9B.**

| GPC MOLECULAR WEIGHT COMPARISONS OF MESO-AND RACEMIC LACTIDE POLYMERS AND COPOLYMERS | | | | | | |
|---|---|---|---|---|---|---|
| Example Nos. | Composition | Res. Mon., % | GPC x 10⁻³ | | | |
| | | | Mₙ | M_{w} | M_{z} | M_{w}/Mₙ |
| 31B | D,L-PLA | -- | 97.5 | 341 | 757 | 3.49 |
| 32B | Meso PLA | 2.76 | 62.5 | 152 | 264 | 2.42 |
| 33B | 90/10, L-/meso | 1.67 | 29 | 142 | 301 | 1.67 |
| 34B | 90/10, L-/D,L | -- | 91.3 | 201 | 350 | 2.20 |

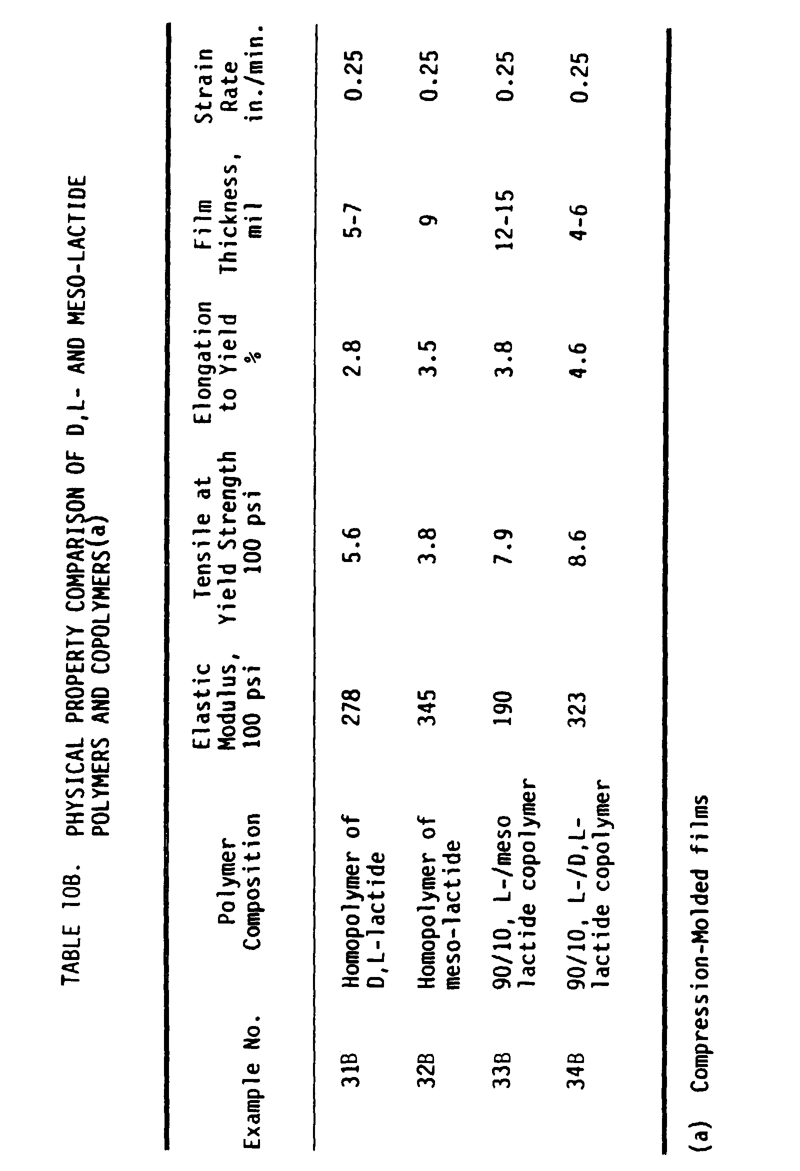

An overall description of the composition prepared in accordance with the invention includes an environmentally biodegradable polymer comprising polymerized lactic acid units of the structure of formula I, where n is an integer between 75 and 10,000 and the alpha carbon is a mixture of L- and D-configurations with a preponderance of either D- or L-units, wherein the polymer is suitable for replacement of polystyrene. The D- and L-units of the polymer may preferably be prepared from L-lactide or D-lactide, at 85 to 95 parts by weight, and D,L-lactide at 15 to 5 parts by weight.

An environmentally biodegradable composition with improved properties more like those of polystyrene comprises blends of a physical mixture of polymerized lactic acid units of the structure of formula I, where n is an integer between 75 and 10,000 and the alpha carbon is a random mixture of L- and D-configurations with a preponderance of either D- or L-units, a lactide hompolymer of D-lactide or L-lactide. Compositions having n equal to an integer between 150 and 10,000 have a good balance between strength and melt processability.

A general description of the process for producing the composition comprises mixing with a catalyst, heating, and melting L-lactide or D-lactide monomer and D,L-lactide monomer whereby the L-lactide monomer or D-lactide monomer is in an amount of 85 to 95 parts by weight and D,L-lactide monomer is in an amount of 15 to 5 parts by weight, to form an intimate solution; polymerizing the solution; and treating the polymer to modify its properties so as to make the polymer suitable as a replacement for polystyrene. The properties of the composition may be adjusted by adding a nucleating agent; adding a D-lactide or L-lactide homopolymer by blending to produce a physical mixture; orienting the polymer; adding a nucleating agent and a D-lactide or L-lactide polymer by blending; adding a nucleating agent and a D-lactide or L-lactide polymer by blending and orienting the polymer; adding chain transfer agents to the polymerization step so as to adjust the characteristics to a polystyrene replacement, annealing at an elevated temperature, and adding additional plasticizer where the plasticizer is selected from D-lactide, L-lactide, meso D,L-lactide, lactic acid, lactide oligomer, lactic acid oligomer, and mixtures thereof. If a monomer is selected as a plasticizer a unique composition may be obtained by adding monomer that is stereochemically different from that used to obtain the polylactide in the composition. Similarly, addition of oligomer stereochemically different from that which may be obtained during polymerization of the polymer gives a unique product. Color bodies can be excluded by performing the polymerization in an inert atmosphere and at reaction temperatures preferably at 140°C or below. Various combinations of the above treatments can be employed to obtain the optimum characteristics as those skilled in the art will appreciate, once knowing the teachings of the invention.

As can be noted in the aforementioned, a higher amount of monomer or oligomer can have significant effect. in the present second embodiment lower amounts of monomer and oligomer are preferred to impart stiffness. Plasticizer present in an amount of 0.1 to 5 percent is preferred. The composition usually contains plasticizer in an amount that depends on polymerization conditions or on the amount added after polymerization. The additional monomer used as plasticizer may be selected from D-lactide, L-lactide, meso D,L-lactide, racemic D,L-lactide, and mixtures thereof. Oligomers of lactide or lactic acid may also be added. Unique compositions may be obtained by addition of monomer or oligomer stereochemically different from those selected for the polymers in the composition.

Further provided by the invention is a method for replacing a thermoplastic composition with the biodegradable composition of the invention where the thermoplastic composition comprises first orientable polystyrene units, by replacing the first polymer units with a second orientable polymer having an unoriented tensile strength of at least 5,000 and a tangent modulus of at least 200,000; wherein the second polymer comprises polylactic acid units of the structure in formula I, wherein n is the number of repeating units and n is an integer, 75 ≤ n ≤ 10,000, and the alpha carbon is a mixture of L- and D- configurations with a preponderance of either D- or L- units, wherein the polymer is prepared from L- or D-lactide at 85 to 95 parts by weight, and D,L-lactide at 15 to 5 parts by weight and is plasticized with a plasticizer selected from lactide, oligomers of lactic acid, oligomers of lactide and mixtures thereof between 0.1 and 5.0 weight percent.

Contemplated equivalents of the compositions of the invention are those that contain minor amounts of other materials. The compositions produced in accordance with the present invention can be modified, if desired, by the addition of a cross-linking agent, other plasticizers, a coloring agent, a filler and the like.

The compositions herein can be processed by melt fabrication into useful articles of manufacture having a self supporting structure such as disposable containers, eating utensils, trays, plates, drinking cups, single serving trays, syringes, medical trays, packaging films and the like. The compositions are useful in that they can have the characteristics of the usual polystyrenes and therefore substitute for them yet degrade in the environment. The compositions are especially useful for articles having only a one time use or a short life span in use before disposal.

While the invention has been described above with reference to various specific examples and embodiments, it will be understood that the invention is not limited to such illustrated examples and embodiments and may be variously practised within the scope of the claims.

## Claims

1. A process for producing an environmentally biodegradable composition of polylactic acid comprising:
a. preparing lactide monomer and catalyst;
b. polymerizing the monomer of step (a) to form a polymer at a temperature sufficiently low to allow the reaction to be stopped prior to complete polymerization;
c. monitoring the level of monomer in step (b); to determine the amount of monomer remaining;
d. stopping the polymerization of step (b) prior to complete reaction at an amount of monomer determined in step (c) so that unreacted monomer in a predetermined amount is trapped in association with the polymer as plasticizer, which is intimately dispersed in the polymer; wherein the predetermined amount of unreacted lactide is from 2 to 30 weight percent ; and
e. incorporating additional plasticizer into the composition whereby the additional plasticizer is further selected from L-lactide, D-lactide, meso D,L-lactide, lactic acid, oligomers of lactide, oligomers of lactic acid, and mixtures thereof; and the additional plasticizer is also intimately dispersed in the polymer.

2. The process of claim 1 whereby the additional platicizer is a monomer sterochemically different from that selected to prepare the polymer.

3. A process for producing a biodegradable composition of polylactic acid comprising:
a. preparing lactide monomer and catalyst;
b. polymerizing the monomer of the solution of step (a) to form a polymer; and
c. incorporating plasticizer into the polymer of step (b), whereby the plasticizer is selected from D-lactide, L-lactide, D,L-lactide, oligomers of lactic acid, oligomers of lactide, and mixtures thereof; and the plasticizer is intimately dispersed within the polymer.

4. A method of plasticizing a homopolymer of any one of L-lactide, D-lactide, D,L-lactide or copolymers thereof or mixtures thereof, characterised in that any one of a lactide monomer, lactic acid, an oligomer of lactide, or an oligomer of lactic acid, or mixtures thereof, is used as the plasticizer.

5. The method of claim 4, wherein a composition resulting from said method comprises a polymer of the formula: 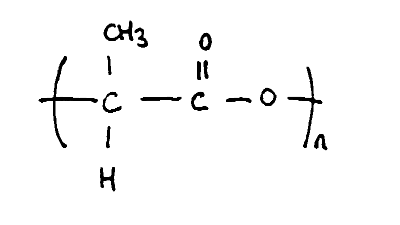 wherein n is the number of repeating units and n is an integer, 150° ≤ n ≤ 20,000, and the unoriented composition has a tensile strength of 2.1 x 10⁵ to 1.4 x 10⁷ kgm⁻² (300 to 20,000 psi), an elongation to failure of 50 to 1,000 percent, and a tangent modulus of 1.4 x 10⁷ to 1.75 x 10⁸ kgm⁻² (20,000 to 250,000 psi); wherein the plasticizer is intimately dispersed in the polymer and wherein the plasticizer comprises from 2 to 60 weight percent of the polymer.

6. The method of claim 4 wherein the oligomers of lactic acid or lactide have the formula: 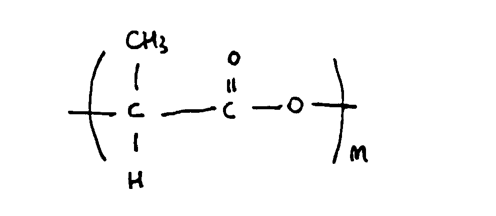 where m is an integer: 2 ≤ m ≤ 75.

7. The method of claim 4 wherein the plasticizer dispersed within the composition is selected from monomers consisting of D-lactide, L-lactide, meso D,L-lactide, racemic D,L-lactide and mixtures thereof such that at least part of the dispersed monomer is stereochemically different from that used to prepare the polymer; wherein the plasticizer is intimately dispersed in the polymer.

8. The method of claim 5 wherein the composition comprises an oligomer intimately dispersed within the composition which is not produced during polymerization of the polymer.

## Patentansprüche

1. Verfahren zur Herstellung einer unter Umweltbedingungen biologisch abbaubaren Zusammensetzung von Polymilchsäure, bei dem man
a) Lactidmonomer und Katalysator bereitet,
b) das Monomer der Stufe (a) unter Bildung eines Polymers bei einer genügend niedrigen Temperatur polymersiert, um zu erlauben, die Reaktion vor vollständiger Polymerisation anzuhalten,
c) den Gehalt an Monomer in der Stufe (b) überwacht, um die Menge an restlichem Monomer zu bestimmen,
d) die Polymerisation der Stufe (b) vor vollständiger Reaktion bei einer Monomermenge, die in Stufe (c) bestimmt wird, anhält, so daß unumgesetztes Monomer in einer vorbestimmten Menge in Verbindung mit dem Polymer als Weichmacher eingeschlossen ist, welcher innig in dem Polymer dispergiert ist, wobei die vorbestimmte Menge an unumgesetztem Lactid 2 bis 30 Gew.% beträgt, und
e) zusätzlichen Weichmacher in die Zusammensetzung einarbeitet, wobei der zusätzliche Weichmacher weiterhin unter L-Lactid, D-Lactid, meso-D,L-Lactid, Milchsäure, Lactidoligomeren, Milchsäureoligomeren und Gemischen hiervon ausgewählt wird, und den zusätzlichen Weichmacher auch innig in dem Polymer dispergiert.

2. Verfahren nach Anspruch 1, bei dem der zusätzliche Weichmacher ein Monomer ist, das sich stereochemisch von jenem unterscheidet, welches zur Herstellung des Polymers ausgewählt wird.

3. Verfahren zur Herstellung einer biologisch abbaubaren Zusammensetzung von Polymilchsäure, bei dem man
a) Lactidmonomer und Katalysator bereitet,
b) das Monomer der Lösung von Stufe (a) unter Bildung eines Polymers polymerisiert und
c) Weichmacher in das Polymer der Stufe (b) einarbeitet, wobei der Weichmacher unter D-Lactid, L-Lactid, D,L-Lactid, Milchsäureoligomeren, Lactidoligomeren und Gemischen hiervon ausgewählt wird, und den Weichmacher innig in dem Polymer dispergiert.

4. Verfahren zum Weichmachen eines Homopolymers von L-Lactid, D-Lactid, D,L-Lactid oder Copolymeren hiervon oder Gemischen hiervon, **dadurch gekennzeichnet**, daß man ein Lactidmonomer, Milchsäure, ein Lactidoligomer oder ein Milchsäureoligomer oder Gemische hiervon als den Weichmacher verwendet.

5. Verfahren nach Anspruch 4, bei dem eine aus dem Verfahren resultierende Zusammensetzung ein Polymer der Formel 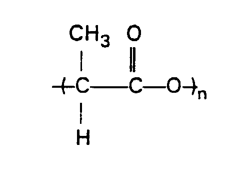 umfaßt, worin n die Anzahl der sich wiederholenden Einheiten ist und n eine ganze Zahl, 150 ≤ n ≤ 20 000, bedeutet und die nichtorientierte Zusammensetzung eine Zugfestigkeit von 2,1 x 10⁵ bis 1,4 x 10⁷ kgm⁻² (300 bis 20 000 psi), eine Dehnung beim Bruch von 50 bis 1000 % und einen Tangensmodul von 1,4 x 10⁷ bis 1,75 x 10⁸ kgm⁻² (20 000 bis 250 000 psi) hat, worin der Weichmacher in dem Polymer innig dispergiert ist und 20 bis 60 Gew.% des Polymers umfaßt.

6. Verfahren nach Anspruch 4, bei dem die Milchsäure- oder Lactidoligomeren die Formel 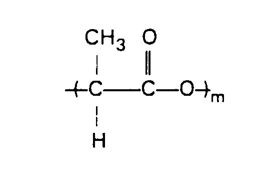 haben, worin m eine ganze Zahl von: 2 ≤ m ≤ 75 ist.

7. Verfahren nach Anspruch 4, bei dem der in der Zusammensetzung dispergierte Weichmacher unter Monomeren ausgewählt wird, die aus D-Lactid, L-Lactid, meso-D,L-Lactid, racemischem D,L-Lactid und Gemischen hiervon bestehen, so daß sich wenigstens ein Teil des dispergierten Monomers stereochemisch von jenem unterscheidet, das zur Herstellung des Polymers verwendet wird, wobei der Weichmacher innig in dem Polymer dispergiert wird.

8. Verfahren nach Anspruch 5, bei dem die Zusammensetzung ein innig in der Zusammensetzung dispergiertes Oligomer umfaßt, welches nicht während der Polymerisation des Polymers erzeugt wird.

## Revendications

1. Procédé de préparation d'une composition d'acide polylactique biodégradable dans l'environnement, comprenant les étapes consistant à :
a. préparer un monomère lactide et un catalyseur ;
b. polymériser le monomère de l'étape (a) pour former un polymère à une température suffisamment basse pour permettre de stopper la réaction avant que la polymérisation soit complète ;
c. surveiller la quantité de monomère dans l'étape (b) pour déterminer la quantité de monomère restant ;
d. stopper la polymérisation de l'étape (b) avant que la réaction soit complète, quand on a atteint une quantité de monomère déterminé dans l'étape (c), de façon à ce que le monomère n'ayant pas subi de réaction, en une quantité prédéterminée, soit enfermé en association avec le polymère en tant que plastifiant, qui est intimement dispersé dans le polymère ; la quantité prédéterminée de lactide n'ayant pas subi de réaction étant de 2 à 30 % en poids ; et
e. incorporer un plastifiant supplémentaire dans la composition, le plastifiant supplémentaire étant choisi parmi le L-lactide, le D-lactide, le méso-D,L-lactide, l'acide lactique, des oligomères de lactide, des oligomères d'acide lactique, et des mélanges de ceux-ci ; et le plastifiant supplémentaire étant aussi dispersé intimement dans le polymère.

2. Procédé selon la revendication 1, dans lequel le plastifiant supplémentaire est un monomère qui, du point de vue de la stéréochimie, est différent de celui choisi pour préparer le polymère.

3. Procédé de préparation d'une composition biodégradable d'acide polylactique, comprenant les étapes consistant à :
a. préparer un monomère lactide et un catalyseur ;
b. polymériser le monomère de la solution de l'étape (a) pour former un polymère ; et
c. incorporer un plastifiant dans le polymère de l'étape (b), ce plastifiant étant choisi parmi le D-lactide, le L-lactide, le D,L-lactide, des oligomères d'acide lactique, des oligomères de lactide, ainsi que des mélanges de ceux-ci ; et le plastifiant étant intimement dispersé à l'intérieur du polymère.

4. Procédé de plastification d'un homopolymère quelconque de L-lactide, de D-lactide, de D,L-lactide ou de copolymères ou de mélanges de ceux-ci, caractérisé en ce qu'on utilise l'un quelconque des composés monomère lactide, acide lactique, oligomère de lactide ou oligomère d'acide lactique, ou des mélanges de ceux-ci, en tant que plastifiant.

5. Procédé selon la revendication 4, dans lequel une composition résultant dudit procédé comprend un polymère ayant la formule : 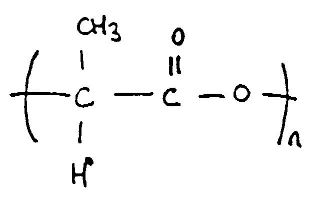 dans laquelle n est le nombre d'unités répétitives et est un nombre entier, 150 ≤ n ≤ 20 000, et la composition non orientée a une résistance à la rupture par traction de 2,1 x 10⁵ à 1,4 x 10⁷ kgm⁻² (300 à 20 000 psi), un allongement à la rupture de 50 à 1 000 % et un module tangent de 1,4 x 10⁷ à 1,75 x 10⁸ kgm⁻² (20 000 à 250 000 psi), le plastifiant étant intimement dispersé dans le polymère, et le plastifiant constituant de 2 à 60 % en poids du polymère.

6. Procédé selon la revendication 4, dans lequel les oligomères d'acide lactique ou de lactide ont la formule : 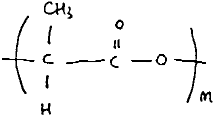 dans laquelle m est un nombre entier : 2 ≤ m ≤ 75.

7. Procédé selon la revendication 4, dans lequel le plastifiant dispersé dans la composition est choisi parmi des monomères constitués de D-lactide, L-lactide, méso-D,L-lactide, D,L-lactide racémique, et des mélanges de ceux-ci, de telle façon qu'au moins une partie du monomère dispersé soit différente, du point de vue stéréochimique, de celui utilisé pour préparer le polymère, le plastifiant étant intimement dispersé dans le polymère.

8. Procédé selon la revendication 5, dans lequel la composition comprend un oligomère intimement dispersé dans la composition, qui n'est pas produit pendant la polymérisation du polymère.
